# EUROPEAN PATENT APPLICATION

(11) **EP 3 085 775 A1**
(43) Date of publication of application: **26.10.2016**
(21) Application number: 15164196.6
(22) Date of filing: 20.04.2015
(51) Int. Cl.: C12N 9/10

(54) **NUCLEIC ACID MOLECULE AND USES THEREOF**

(71) Applicant: Universität für Bodenkultur Wien, 1180 Wien (AT)
(72) Inventor: Loos, Andreas, 1120 Wien (AT); Steinkellner, Hertha, 1190 Wien (AT); Mach, Lukas, 2103 Langenzersdorf (AT)
(74) Representative: KLIMENT & HENHAPEL

(57) **Abstract**

The present invention relates to a nucleic acid molecule encoding
a) a modified tyrosylprotein sulfotransferase of a wild-type tyrosylprotein sulfotransferase, wherein the cytoplasmic transmembrane stem (CTS) region of the wild-type tyrosylprotein sulfotransferase is replaced by a heterologous CTS region, or
b) a fusion protein comprising a catalytically active fragment of a tyrosylprotein sulfotransferase fused to a heterologous CTS region.

## Description

The present invention relates to means and methods for producing sulfated polypeptides in plants.

Most proteins, in particular in eukaryotic systems, undergo posttranslational modifications. Such modifications are important because they may alter physical and chemical properties, conformation, distribution, stability, activity, folding and consequently, function of the proteins. Moreover, many proteins show (significant) activity only if they are posttranslationally modified. Higher eukaryotes perform a variety of post-translational modifications like methylation, sulfation, phosphorylation, lipid addition and glycosylation. Secreted proteins, membrane proteins and proteins targeted to vesicles or certain intracellular organelles are likely to be glycosylated. The most common glycosylation is N-linked glycosylation where oligosaccharides are added to asparagine residues found in Asn-X-Ser/Thr sequences of proteins. Another type of glycosylation is 0-linked glycosylation which involves simple oligosaccharide chains or glycosaminoglycan chains. Sulfation is known to play an important role in strengthening protein-protein interactions. Types of human proteins which often undergo sulfation include adhesion molecules, G-protein-coupled receptors, coagulation factors, serine protease inhibitors, extracellular matrix proteins and hormones. The target amino acid of the sulfation reaction is tyrosine and the reaction is, thus, called tyrosine sulfation. In the course of the sulfation reaction a sulfate group is added to a tyrosine residue of a target protein molecule. Sulfation is catalyzed by tyrosylprotein sulfotransferase (TPST) in the Golgi apparatus of eukaryotic cells.

As mentioned above posttranslational modifications influence or are often essential for the biological activity of polypeptides and proteins. This has to be considered when host cells are selected to be used for the recombinant production of polypeptides and proteins. Especially the recombinant expression of animal polypeptides and proteins in plant or yeast cells often requires a modification of the host cell to adapt the posttranslational modification to the polypeptides and proteins to be expressed. Plant cells, for instance, have already been modified to recombinantly produce proteins having an animal like glycosylation. However, strategies to sulfate recombinantly produced proteins of animal origin in plant cells, for instance, have not been established yet.

It is therefore an object of the present invention to provide methods and means enabling a host cell, preferably of non-animal origin, to sulfate a recombinant protein preferably derived from an animal.

The present invention relates to a nucleic acid molecule encoding
a) a modified tyrosylprotein sulfotransferase of a wild-type tyrosylprotein sulfotransferase, wherein the cytoplasmic transmembrane stem (CTS) region of the wild-type tyrosylprotein sulfotransferase is replaced by a heterologous CTS region, or
b) a fusion protein comprising a catalytically active fragment of a tyrosylprotein sulfotransferase fused to a heterologous CTS region.

It turned out that the replacement of a naturally occurring CTS region of a tyrosylprotein sulfotransferase by a CTS region of another polypeptide or protein results in an increase of the protein sulfation rate in a host cell expressing such a modified tyrosylprotein sulfotransferase. Furthermore, the recombinant expression of the modified tyrosylprotein sulfotransferase of the present invention enables host cells to sulfate recombinant proteins and polypeptides although the corresponding wild-type host cells do not have such sulfation activities.

"Modified tyrosylprotein sulfotransferase", as used herein, refers to a tyrosylprotein sulfotransferase which has a different amino acid sequence as naturally occurring tyrosylprotein sulfotransferases ("wild-type tyrosylprotein sulfotransferases"). These modified tyrosylprotein sulfotransferases comprise instead of their naturally occurring CTS region a heterologous CTS region.

As used herein, a "cytoplasmic transmembrane stem (CTS) region" and a "CTS region" or a "cytoplasmic transmembrane stem (CTS) domain" and a "CTS domain" comprises the cytoplasmic tail, transmembrane domain and stem region of Golgi-resided proteins and polypeptides. CTS regions mediate sorting of the proteins and polypeptides attached thereto into the different functional compartments of the Golgi apparatus.

CTS regions of Golgi-resided proteins can be identified using methods well-known in the art, such as, for example, hydropathy plot analysis and sequence alignments with known CTS regions. A CTS region may consist of a substantial part of a CTS region, such as at least 50% or at least 60% or at least 70% or at least 80% or at least 90% of a CTS region. The CTS region/domain may consist of 1 to 100, preferably 5 to 90, more preferably 10 to 80, more preferably 15 to 70, more preferably 15 to 60, more preferably 20 to 50, more preferably 25 to 45, more preferably 30 to 40, amino acid residues located at the Cor N-terminus of a Golgi-resided protein or polypeptide.

The term "replaced by", as used herein, means that the cytoplasmic transmembrane stem (CTS) region of the wild-type tyrosylprotein sulfotransferase is at least partially, preferably entirely, exchanged by a heterologous CTS region, whereby "heterologous" means that the CTS region is not naturally occurring in said wild-type tyrosylprotein sulfotransferase.

"Fusion protein" as used herein refers to a fusion of two or more amino acid sequences that are not naturally linked together. The fusion of the amino acid sequences can either be made by chemically linking ex vivo synthesized amino acid sequences. In an embodiment, the amino acid sequences are an in-frame translational fusion, i.e. correspond to a recombinant molecule expressed from a nucleic acid sequence in a prokaryotic or eukaryotic expression system. The term "fusion protein" as used herein refers also to a protein which may be created through genetic engineering from two or more proteins/peptides coding sequences joined together in a single coding sequence. In general, this is achieved by creating a "fusion gene", a nucleic acid that encodes and expresses the fusion protein. For example, a fusion gene that encodes a fusion protein may be made by removing the stop codon from a first DNA sequence encoding the first protein, then appending a DNA sequence encoding the second protein in frame. The resulting fusion gene sequence will then be expressed by a cell as a single fusion protein. Fusion proteins may include a linker (or "spacer") sequence which can promote appropriate folding and activity of each domain of the fusion protein.

"Catalytically active fragment" or "enzymatically active fragment", as used herein, refers to a polypeptide fragment that contains the catalytically active domain of a tyrosylprotein sulfotransferase sufficient to exhibit activity. A catalytically active fragment is the portion of a tyrosylprotein sulfotransferase that, under appropriate conditions, can exhibit catalytic activity and is able to transfer a sulfate residue to a tyrosyl residue of a peptide, polypeptide or protein. Typically, a catalytically active fragment is a contiguous sequence of amino acid residues of a tyrosylprotein sulfotransferase that contains the catalytic domain and required portions for recognizing the substrate to be sulfated. A preferred enzymatically/catalytically active fragment of a tyrosylprotein sulfotransferase lacks amino acid residues 1 to 100, preferably 5 to 90, more preferably 10 to 80, more preferably 15 to 70, more preferably 15 to 60, more preferably 20 to 50, more preferably 25 to 45, more preferably 30 to 40, even more preferably 1 to 39, of a wild-type tyrosylprotein sulfotransferase (e.g. SEQ ID No. 1).

"Nucleic acid", as used herein, refers to a deoxyribonucleotide (DNA) or a ribonucleotide polymer (RNA) in either single- or double-stranded form. The nucleic acid molecule of the present invention is preferably a DNA molecule.

The modified tyrosylprotein sulfotransferase and the fusion protein according to the present invention comprise a heterologous CTS region, i.e. a CTS region which is not naturally occurring in the corresponding wild-type tyrosylprotein sulfotransferase. This heterologous CTS region is preferably a CTS region of a protein or polypeptide of a plant or animal.

According to a preferred embodiment of the present invention the plant CTS region is a CTS region of a protein or polypeptide of *Arabidopsis thaliana, Nicotiana spp, Physcomitrella patens or medicago truncatula.*

In a further embodiment of the present invention animal CTS region is a mammalian CTS region, preferably of a protein or polypeptide of a rat, more preferably of a protein or polypeptide of *Rattus norvegicus,* or of human origin.

The heterologous CTS region is preferably a CTS region of a glycosyltransferase or a tyrosylprotein sulfotransferase.

According to a preferred embodiment of the present invention the glycosyltransferase is selected from the group consisting of a fucosyltransferase, preferably an α1,3-fucosyltransferase, more preferably an α1,3-Fucosyltransferase 11, and a sialytransferase, preferably an α2,6-sialytransferase.

According to a further preferred embodiment of the present invention the tyrosylprotein sulfotransferase is a plant or animal tyrosylprotein sulfotransferase.

According to another preferred embodiment of the present invention the animal tyrosylprotein sulfotransferase is a mammalian, preferably human or mouse, tyrosylprotein sulfotransferase, a nematode, preferably a *Caenorhabditis elegans,* tyrosylprotein sulfotransferase or an insect, preferably a *Drosophila melanogaster,* tyrosylprotein sulfotransferase.

The plant tyrosylprotein sulfotransferase is preferably a *Arabidopsis thaliana* tyrosylprotein sulfotransferase.

The heterologous CTS region can be fused either N- or C-terminally to a catalytically active fragment of a tyrosylprotein sulfotransferase.

According to a preferred embodiment of the present invention the wild-type tyrosylprotein sulfotransferase comprises or consists of an amino acid sequence selected from the group consisting of SEQ ID No. 1, SEQ ID No. 13, SEQ ID No. 15, SEQ ID No. 16, SEQ ID No. 19, SEQ ID No. 20, SEQ ID No. 23 and SEQ ID No. 25.

According to a further preferred embodiment of the present invention the wild-type tyrosylprotein sulfotransferase is encoded by a nucleic acid sequence selected from the group consisting of SEQ ID No. 2, SEQ ID No. 14, SEQ ID No. 17, SEQ ID No. 18, SEQ ID No. 21, SEQ ID No. 22, SEQ ID No. 24 and SEQ ID No. 26.

Particularly preferred is a human wild-type tyrosylprotein sulfotransferase comprising amino acid sequence SEQ ID No. 1 or 13, whereby amino acid residues 1 to 50, preferably 1 to 45, more preferably 1 to 42, more preferably 1 to 41, more preferably 1 to 40, more preferably 1 to 38, more preferably 1 to 37, more preferably 1 to 35, more preferably 1 to 30, in particular 1 to 39, of SEQ ID No. 1 and amino acid residues 1 to 50, preferably 1 to 45, more preferably 1 to 42, more preferably 1 to 41, more preferably 1 to 39, more preferably 1 to 38, more preferably 1 to 35, more preferably 1 to 30, in particular 1 to 40, of SEQ ID No. 13 represent the CTS region:

MVGKLKQNLLLACLVISSVTVFYLGQHAMECHHRIEERSQPVKLESTRTTVRTGLDLKAN
KTFAYHKDMPLIFIGGVPRSGTTLMRAMLDAHPDIRCGEETRVIPRILALKQMWSRSSKE
KIRLDEAGVTDEVLDSAMQAFLLEIIVKHGEPAPYLCNKDPFALKSLTYLSRLFPNAKFL
LMVRDGRASVHSMISRKVTIAGFDLNSYRDCLTKWNRAIETMYNQCMEVGYKKCMLVHYE
QLVLHPERWMRTLLKFLQIPWNHSVLHHEEMIGKAGGVSLSKVERSTDQVIKPVNVGALS

KWVGKIPPDVLQDMAVIAPMLAKLGYDPYANPPNYGKPDPKIIENTRRVYKGEFQLPDFL
KEKPQTEQVE (SEQ ID No. 1)

MRLSVRRVLLAAGCALVLVLAVQLGQQVLECRAVLAGLRSPRGAMRPEQEELVMVGTNHV
EYRYGKAMPLIFVGGVPRSGTTLMRAMLDAHPEVRCGEETRIIPRVLAMRQAWSKSGREK
LRLDEAGVTDEVLDAAMQAFILEVIAKHGEPARVLCNKDPFTLKSSVYLSRLFPNSKFLL
MVRDGRASVHSMITRKVTIAGFDLSSYRDCLTKWNKAIEVMYAQCMEVGKEKCLPVYYEQ
LVLHPRRSLKLILDFLGIAWSDAVLHHEDLIGKPGGVSLSKIERSTDQVIKPVNLEALSK
WTGHIPGDVVRDMAQIAPMLAQLGYDPYANPPNYGNPDPFVINNTQRVLKGDYKTPANLK
GYFQVNQNSTSSHLGSS (SEQ ID No. 13)

A nucleic acid sequence encoding the human wild-type tyrosylprotein sulfotransferase may comprise SEQ ID No. 2 or 14, whereby nucleotide residues 1 to 150, preferably 1 to 135, more preferably 1 to 126, more preferably 1 to 123, more preferably 1 to 120, more preferably 1 to 114, more preferably 1 to 111, more preferably 1 to 105, more preferably 1 to 90, in particular 1 to 117, of SEQ ID No. 2 and nucleotide residues 1 to 150, preferably 1 to 135, more preferably 1 to 126, more preferably 1 to 123, more preferably 1 to 117, more preferably 1 to 114, more preferably 1 to 105, more preferably 1 to 90, in particular 1 to 120, of SEQ ID No. 14 encode the CTS region:

atggttggaaagctgaagcagaacttactattggcatgtctggtgattagttctgtgact
gtgttttacctgggccagcatgccatggaatgccatcaccggatagaggaacgtagccag
ccagtcaaattggagagcacaaggaccactgtgagaactggcctggacctcaaagccaac
aaaacctttgcctatcacaaagatatgcctttaatatttattggaggtgtgcctcggagt
ggaaccacactcatgagggccatgctggacgcacatcctgacattcgctgtggagaggaa
accagggtcattccccgaatcctggccctgaagcagatgtggtcacggtcaagtaaagag
aagatccgcctggatgaggctggtgttactgatgaagtgctggattctgccatgcaagcc
ttcttactagaaattatcgttaagcatggggagccagccccttatttatgtaataaagat
ccttttgccctgaaatctttaacttacctttctaggttattccccaatgccaaatttctc
ctgatggtccgagatggccgggcatcagtacattcaatgatttctcgaaaagttactata
gctggatttgatctgaacagctataggactgtttgacaaagtggaatcgtgctatagag
accatgtataaccagtgtatggaggttggttataaaaagtgcatgttggttcactatgaa
caacttgtcttacatcctgaacggtggatgagaacactcttaaagttcctccagattcca
tggaaccactcagtattgcaccatgaagagatgattggaaagctggggggagtgtctctg
tcaaaagtggagagatctacagaccaagtaatcaagccagtcaatgtaggagctctatca
aaatgggttgggaagataccgccagatgttttacaagacatggcagtgattgctcctatg
cttgccaagcttggatatgacccatatgccaacccacctaactacggaaaacctgatccc

aaaattattgaaaacactcgaagggtctataagggagaattccaactacctgactttctt
aaagaaaaaccacagactgagcaagtggagtag (SEQ ID No. 2)

atgcgcctgtcggtgcggagggtgctgctggcagccggctgcgccctggtcctggtgctg
gcggttcagctgggacagcaggtgctagagtgccggcggtgctggcggcctgcggagc
ccccgggggccatgcggcctgagcaggaggagctggtgatggtgggcaccaaccacgtg
gaataccgctatggcaaggccatgccgctcatcttcgtgggtggcgtgcctcgcagtggc
accacgttgatgcgcgccatgctggacgcccaccccgaggtgcgctgcggcgaggagacc
cgcatcatcccgcgcgtgctggccatgcgccaggcctggtccaagtctggccgtgagaag
ctgcggctggatgaggcgggggtgacggatgaggtgctggacgccgccatgcaggccttc
atcctggaggtgattgccaagcacggagagccggcccgcgtgctctgcaacaaggaccca
tttacgctcaagtcctcggtctacctgtcgcgcctgttccccaactccaagttcctgctg
atggtgcgggacggccgggcctccgtgcactccatgatcacgcgcaaagtcaccattgcg
ggctttgacctcagcagctaccgtgactgcctcaccaagtggaacaaggccatcgaggtg
atgtacgcccagtgcatggaggtaggcaaggagaagtgcttgcctgtgtactacgagcag
ctggtgctgcacccccaggcgctcactcaagctcatcctcgacttcctcggcatcgcctgg
agcgacgctgtcctccaccatgaagacctcattggcaagcccggtggtgtctccctgtcc
aagatcgagcggtccacggaccaggtcatcaagcctgttaacctggaagcgctctccaag
tggactggccacatccctggggatgtggtgcgggacatggcccagatcgcccccatgctg
gctcagctcggctatgacccttatgcaaacccccccaactatggcaaccctgaccccttc
gtcatcaacaacacacagcgggtcttgaaaggggactataaaacaccagccaatctgaaa
ggatattttcaggtgaaccagaacagcacctcctcccacttaggaagctcgtgatttcca
gatctccgcaaatgacttcattgccaagaagagaagaaaatgcatttaa (SEQ ID
No. 14)

Particularly preferred is a mammalian tyrosylprotein sulfotransferase of a mouse comprising amino acid sequence SEQ ID No. 15 or SEQ ID No. 16, whereby amino acid residues 1 to 50, preferably 1 to 45, more preferably 1 to 42, more preferably 1 to 41, more preferably 1 to 40, more preferably 1 to 38, more preferably 1 to 37, more preferably 1 to 35, more preferably 1 to 30, in particular 1 to 39, of SEQ ID No. 15 and amino acid residues 1 to 65, preferably 1 to 60, more preferably 1 to 55, more preferably 1 to 54, more preferably 1 to 52, more preferably 1 to 50, more preferably 1 to 45, more preferably 1 to 40, in particular 1 to 53, of SEQ ID No. 16 represent the CTS region:

MVGKLKQNLLLACLVISSVTVFYLGQHAMECHHRIEERSQPARLENPKATVRAGLDIKAN
KTFTYHKDMPLIFIGGVPRSGTTLMRAMLDAHPDIRCGEETRVIPRILALKQMWSRSSKE
KIRLDEAGVTDEVLDSAMQAFLLEVIVKHGEPAPYLCNKDPFALKSLTYLARLFPNAKFL

LMVRDGRASVHSMISRKVTIAGFDLNSYRDCLTKWNRAIETMYNQCMEVGYKKCMLVHYE
QLVLHPERWMRTLLKFLHIPWNHSVLHHEEMIGKAGGVSLSKVERSTDQVIKPVNVGALS
KWVGKIPPDVLQDMAVIAPMLAKLGYDPYANPPNYGKPDPKILENTRRVYKGEFQLPDFL
KEKPQTEQVE (SEQ ID No. 15)

MRRAPWLGLRPWLGMRLSVRKVLLAAGCALALVLAVQLGQQVLECRAVLGGTRNPRRMRP
EQEELVMLGADHVEYRYGKAMPLIFVGGVPRSGTTLMRAMLDAHPEVRCGEETRIIPRVL
AMRQAWTKSGREKLRLDEAGVTDEVLDAAMQAFILEVIAKHGEPARVLCNKDPFTLKSSV
YLARLFPNSKFLLMVRDGRASVHSMITRKVTIAGFDLSSYRDCLTKWNKAIEVMYAQCME
VGRDKCLPVYYEQLVLHPRRSLKRILDFLGIAWSDTVLHHEDLIGKPGGVSLSKIERSTD
QVIKPVNLEALSKWTGHIPRDVVRDMAQIAPMLARLGYDPYANPPNYGNPDPIVINNTHR
VLKGDYKTPANLKGYFQVNQNSTSPHLGSS (SEQ ID No. 16)

A nucleic acid sequence encoding the mammalian, preferably mouse, tyrosylprotein sulfotransferase may comprise SEQ ID No. 17 or SEQ ID No. 18, whereby nucleotide residues 1 to 150, preferably 1 to 135, more preferably 1 to 126, more preferably 1 to 123, more preferably 1 to 120, more preferably 1 to 114, more preferably 1 to 111, more preferably 1 to 105, more preferably 1 to 90, in particular 1 to 117, of SEQ ID No. 17 and nucleotide residues 1 to 195, preferably 1 to 180, more preferably 1 to 165, more preferably 1 to 162, more preferably 1 to 156, more preferably 1 to 150, more preferably 1 to 135, more preferably 1 to 120, in particular 1 to 159, of SEQ ID No. 18 encode the CTS region:

atggttggaagctgaagcagaacttactcttggcgtgtctggtgattagttctgtgacc
gtgttttacctgggccagcatgccatggagtgccatcaccgaatagaggaacgtagccag
ccagcccgactggagaaccccaaggcgactgtgcgagctggcctcgacatcaaagccaac
aaaacattcacctatcacaaagatatgcctttaatattcatcgggggtgtgcctcggagc
ggcaccacactcatgagggctatgctggacgcacatcctgacatccgctgtggagaggaa
accagggtcatccctcgaatcctggccctgaagcagatgtggtcccggtccagtaaagag
aagatccgcttggatgaggcgggtgtcacagatgaagtgctagattctgccatgcaagcc
ttccttctggaggtcattgttaaacatggggagccggcaccttatttatgtaacaaagat
ccgtttgccctgaaatccttgacttaccttgctaggttatttcccaatgccaaatttctc
ctgatggtccgagatggccgggcgtcagtacattcaatgatttctcggaaagttactata
gctggctttgacctgaacagctaccggactgtctgaccaagtggaaccggccatagaa
accatgtacaaccagtgtatggaagttggttataagaaatgcatgttggttcactatgaa
cagctcgtcttacaccctgaacggtggatgagaacgctcttaaagttcctccatattcca
tggaaccattccgttttgcaccatgaagaaatgatcggaaagctgggggagtttctctg
tcaaaggtggaaagatcaacagaccaagtcatcaaacccgtcaacgtggggggcgctatcg

aagtgggttggaagataccccggacgtcttacaagacatggccgtgattgcacccatg
ctcgccaagcttggatatgacccatacgccaatcctcctaactacggaaacctgacccc
aagatccttgaaaacaccaggagggtctataaaggagaatttcagctccctgactttctg
aaagaaaaaccccagacggagcaagtggagtaa (SEQ ID No. 17)

atgaggcgggcccctggctgggcctgcgaccctggctgggcatgcgcctgtcggtgcgt
aaggtgctgctggccgccggctgtgctctggccctggtgctcgctgtgcagcttgggcag
caagtactggagtgccgggcggtgctcgggggcacacggaacccacggaggatgcggccg
gagcaggaggaactggtgatgctcggcgccgaccacgtggagtaccgctatggcaaggcc
atgccactcatctttgtgggcggcgtgccacgcagtggcaccacgctcatgcgcgccatg
ttggacgcacacccagaggtgcgctgtggggaggagacgcgcatcatccctcgtgtgctg
gccatgcggcaggcctggaccaagtctggccgtgagaagctgcggctggacgaggcaggt
gtgacggatgaggtgctggacgcggccatgcaggccttcattctggaggtgatcgccaag
cacggcgaaccagcccgcgtgctgtgtaacaaggaccccttcacactcaagtcatccgtc
tacctggcacgcctgttccccaactccaaattcctgctaatggtgcgtgacggccgggcg
tccgtgcactccatgatcacgcgcaaggtcaccatcgcgggctttgacctcagcagctac
cgagactgcctcaccaagtggaacaaggccatcgaggtgatgtacgcacagtgcatggag
gtgggcagggacaagtgcctgcccgtgtactatgagcagttggtgctgcacccccggcgc
tcactcaaacgcatcctggacttcctgggcatcgcctggagtgacacagtcctgcaccac
gaggacctcattggcaagcctgggggcgtctccttgtccaagatcgagcggtccacggac
caggtcatcaaaccggtgaacttggaagctctctccaagtggacgggccacatccctaga
gacgtggtgagggatatggcccagattgcccccatgctggcccggcttggctatgacccg
tatgcgaatccacccaactatggaaccccgaccccattgtcatcaacaacacacaccgg
gtcttgaaaggagactataaaacgccagccaatctgaaaggatatttcaggtgaaccag
aacagcacctccccacacctaggaagttcgtga (SEQ ID No. 18)

Particularly preferred is a nematode tyrosylprotein sulfotransferase of *Caenorhabditis elegans* comprising amino acid sequence SEQ ID No. 19 or SEQ ID No. 20, whereby amino acid residues 1 to 50, preferably 1 to 45, more preferably 1 to 42, more preferably 1 to 41, more preferably 1 to 40, more preferably 1 to 38, more preferably 1 to 37, more preferably 1 to 35, more preferably 1 to 30, in particular 1 to 39, of SEQ ID No. 19 and amino acid residues 1 to 50, preferably 1 to 45, more preferably 1 to 42, more preferably 1 to 40, more preferably 1 to 38, more preferably 1 to 35, more preferably 1 to 30, of SEQ ID No. 20 represent the CTS region:

MRKNRELLLVLFLVVFILFYFITARTADDPYYSNHREKFNGAAADDGDESLPFHQLTSVR
SDDGYNRTSPFIFIGGVPRSGTTLMRAMLDAHPEVRCGEETRVIPRILNLRSQWKKSEKE
WNRLQQAGVTGEVINNAISSFIMEIMVGHGDRAPRLCNKDPFTMKSAVYLKELFPNAKYL

LMIRDGRATVNSIISRKVTITGFDLNDFRQCMTKWNAAIQIMVDQCESVGEKNCLKVYYE
QLVLHPEAQMRRITEFLDIPWDDKVLHHEQLIGKDISLSNVERSSDQVVKPVNLDALIKW
VGTIPEDVVADMDSVAPMLRRLGYDPNANPPNYGKPDELVAKKTEDVHKNGAEWYKKAVQ
VVNDPGRVDKPIVDNEVSKL (SEQ ID No. 19)

MRAILDAHPDVRCGGETMLLPSFLTWQAGWRNDWVNNSGITQEVFDDAVSAFITEIVAKH
SELAPRLCNKDPYTALWLPTIRRLYPNAKFILMIRDARAVVHSMIERKVPVAGYNTSDEI
SMFVQWNQELRKMTFQCNNAPGQCIKVYYERLIQKPAEEILRITNFLDLPFSQQMLRHQD
LIGDEVDLNDQEFSASQVKNSINTKALTSWFDCFSEETLRKLDDVAPFLGILGYDTSISK
PDYSTFADDDFYQFKNFYS (SEQ ID No. 20)

A nucleic acid sequence encoding the nematode, preferably *Caenorhabditis elegans,* tyrosylprotein sulfotransferase may comprise SEQ ID No. 21 or SEQ ID No. 22, whereby nucleotide residues 1 to 150, preferably 1 to 135, more preferably 1 to 126, more preferably 1 to 123, more preferably 1 to 120, more preferably 1 to 114, more preferably 1 to 111, more preferably 1 to 105, more preferably 1 to 90, in particular 1 to 117, of SEQ ID No. 21 and nucleotide residues 1 to 150, preferably 1 to 135, more preferably 1 to 126, more preferably 1 to 120, more preferably 1 to 114, more preferably 1 to 105, more preferably 1 to 90, of SEQ ID No. 22 encode the CTS region:

atgagaaaaatcgagagttgctactcgtcctcttcctcgtcgttttatactattctat
tttattactgcgagaactgcagacgacccgtactacagtaaccatcggagaaattcaat
ggtgccgccgccgacgacggcgacgagtcgttaccttttcatcaattaacgtcagtacga
agtgatgatggatacaatagaacgtctcctttcatattcataggtggtgttcctcgctcc
ggtacaactctgatgcgtgcgatgcttgacgctcatccagaagtcagatgtggtgaggag
acacgtgtcattccacgcatcctgaatctacggtcacaatggaaaagtcggaaaaggag
tggaatcgactgcagcaggctggagtgacgggtgaagtgattaacaatgcgatcagctcg
tttatcatggagataatggttggccacggagatcggctcctcgtctctgcaacaaggat
ccattcacaatgaaatcagccgtctacctaaaagaactcttcccaaatgccaaatatctt
ctaatgatccgtgatggacggccaccgtgaatagtataatctcacgaaaagtcacaatt
accggattcgatttgaacgatttccgtcaatgcatgacgaaatggaatgcggcaattcaa
ataatggtagatcagtgtgaatcggttggagagaaaaattgtttgaaagtgtattatgag
cagctggtgctacatccggaagcacaaatgcggcgaattacagagttttggatattccg
tgggatgataaagtgctgcaccatgagcagcttattggaaaagatatttctttatcgaat
gtggaacggagctcggatcaagtcgttaaaccggttaatcttgatgctcttatcaaatgg
gttggaacgattcctgaggatgttgttgctgatatggattcggttgcgccgatgttaagg
agattaggatatgatccgaatgcaaatccaccaaactatggaaacccgacgaactagtc
gcgaaaaaaacggaagatgttcataaaaatggagccgaatggtacaagaaagcagttcaa

gtggtcaacgatcccggccgcgtcgataaaccaattgttgataatgaagtatcgaaatta
tag (SEQ ID No. 21)

atgagagctattctagatgcacatccggatgttcgatgtggcggtgaaaccatgctgctt
ccaagtttccttacatggcaagcaggctggcggaatgattgggtcaataattcaggaatt
actcaggaagtatttgacgacgctgtttcagcattcatcactgagatagtcgcgaagcac
agtgaactagcacctcgtctgtgcaacaaggatccatacaccgcattgtggcttccgact
attcgccgactgtacccgaatgcaaagtttattctgatgattcgagatgctcgtgccgta
gttcattcaatgatagaaagaaaagtaccagttgctgggtataatacgtctgatgaaatt
tcaatgtttgttcagtggaatcaggagcttcgaaaaatgacttttcaatgcaataatgcg
ccagggcaatgcataaaagtatattatgaacgactgattcaaaaacctgcggaagaaatc
ctacgtatcaccaacttcctggatctgccattttcccagcaaatgctaagacatcaagat
ttaattggagacgaagttgatttaaacgatcaagaattctctgcatcacaagttaaaaac
tcgataaacactaaagccttaacctcgtggtttgattgttttagtgaagaaactctacga
aaacttgatgacgtggcacctttttgggaattcttggatacgatacgtcgatttcaaaa
cccgattattccacatttgcggatgacgattttaccaatttaaaatttttattcttaa
(SEQ ID No. 22)

Particularly preferred is an insect tyrosylprotein sulfotransferase of *Drosophila melanogaster* comprising amino acid sequence SEQ ID No. 23, whereby amino acid residues 1 to 60, preferably 1 to 55, more preferably 1 to 50, more preferably 1 to 49, more preferably 1 to 48, more preferably 1 to 46, more preferably 1 to 45, more preferably 1 to 40, more preferably 1 to 35, in particular 1 to 47, of SEQ ID No. 23 represent the CTS region:

MRLPYRNKKVTLWVLFGIIVITMFLFKFTELRPTCLFKVDAANELSSQMVRVEKYLTDDN
QRVYSYNREMPLIFIGGVPRSGTTLMRAMLDAHPDVRCGQETRVIPRILQLRSHWLKSEK
ESLRLQEAGITKEVMNSAIAQFCLEIIAKHGEPAPRLCNKDPLTLKMGSYVIELFPNAKF
LFMVRDGRATVHSIISRKVTITGFDLSSYRQCMQKWNHAIEVMHEQCRDIGKDRCMMVYY
EQLVLHPEEWMRKILKFLDVPWNDAVLHHEEFINKPNGVPLSKVERSSDQVIKPVNLEAM
SKWVGQIPGDVVRDMADIAPMLSVLGYDPYANPPDYGKPDAWVQDNTSKLKANRMLWESK
AKQVLQMSSSEDDNTNTIINNSNNKDNNNNQYTINKIIPEQHSRQRQHVQQHLQQQQQ
HLQQQHQRQQQQQREEESESEREAEPDREQQLLHQKPKDVITIKQLPLAGSNNNNINN
NINNNNNNNNIMEDPMADT (SEQ ID No. 23)

A nucleic acid sequence encoding the insect, preferably *Drosophila melanogaster,* tyrosylprotein sulfotransferase may comprise SEQ ID No. 24, whereby nucleotide residues 1 to 180, preferably 1 to 165, more preferably 1 to 150, more preferably 1 to 147, more preferably 1 to 144, more preferably 1 to 138, more preferably 1 to 135, more preferably 1 to 120, more preferably 1 to 105, in particular 1 to 141, of SEQ ID No. 24 encode the CTS region:

atgcgactgccatatcgaaataagaaggtcaccctgtgggtgctcttcggcatcatcgtc
atcaccatgttcctattcaaattcaccgaactgcggcccacatgcctcttcaaggtggac
gccgccaacgagctctcctcccaaatggttcgcgttgagaaatacctcacagatgacaat
caacgcgtttattcatacaaccgtgagatgccattaatattcataggcggcgtgccgaga
tctggacgactttgatgcgcgccatgctggatgcccatcccgatgtgcgctgcggcag
gaaacccgtgtcattccgcgcatcctgcagctgcgctcgcactggctgaagtccgagaag
gagtcgctccgcctgcaggaggccggcatcaccaaagaggtcatgaacagtgccatcgcg
cagttctgtctggaaatcatcgccaaacacggcgagccggcgccgcgcttatgcaacaag
gatccgctgacgctgaaaatgggctcctatgtcatcgagctatttccgaacgctaaattc
ctattcatggtgcgcgacggccgggcgacagttcattcgattatatcgcgcaaggtgaca
atcaccggcttcgatttgagcagctaccggcagtgcatgcagaagtggaaccacgccatc
gaggtgatgcacgagcagtgccggacatcggcaaggaccgctgcatgatggtttactat
gagcagctggtactgcatcccgaggagtggatgcgaaagatactgaaattcctggacgtg
ccatggaacgatgcggtgctgcaccacgaggagttcataaataaaccgaacggtgtgcct
ctgtccaaggtggaacgttcgtcggaccaggttatcaagccggttaatctggaggcgatg
tccaaatgggttggccaaatacccggcgacgtggtgcgcgacatggccgacatagcgccc
atgctgtccgtgctcggctacgatccgtacgcgaatccgccggactatggtaagccagat
gcatgggtgcaggacaacacgtcgaagttaaaggccaatcgaatgctgtgggagagtaag
gcgaagcaagtgctgcagatgtcatccagcgaggatgacaacacgaacaccatcatcaac
aatagcaacaataaggataacaacaataatcagtacacaatcaataaaattataccagaa
caacacagcagacagcggcaacatgtacagcagcaacatctgcagcagcagcagcagcag
catctgcaacagcagcaacatcagcggcagcagcaacagcagcaacgtgaggaggagagc
gagtcggaaaggaagcggaaccggatcgagaacaacaattgttgcatcaaagccaaag
gatgtcattacgataaagcagctgccattagctgggagcaacaataacaacatcaacaat
aacatcaacaacaacaacaacaacaacaacatcatggaggaccccatggcggatacatga
(SEQ ID No. 24)

Particularly preferred is a plant tyrosylprotein sulfotransferase of *Arabidopsis thaliana* comprising amino acid sequence SEQ ID No. 25, whereby amino acid residues 1 to 24 of SEQ ID No. 25 represent a signal sequence and amino acid residues 450 to 500, preferably 460 to 500, more preferably 465 to 500, more preferably 469 to 500, more preferably 470 to 500, in particular 471 to 500, of SEQ ID No. 25 represent the TS region (transmembrane domain with cytosolic tail; see e.g. Moore, PNAS 106 (2009) : 14741-2) :

MQMNSVWKLSLGLLLLSSVIGSFAELDFGHCETLVKKWADSSSSREEHVNKDKRSLKDLL
FFLHVPRTGGRTYFHCFLRKLYDSSEECPRSYDKLHFNPRKEKCKLLATHDDYSLMAKLP
RERTSVMTIVRDPIARVLSTYEFSVEVAARFLVHPNLTSASRMSSRIRKSNVISTLDIWP
WKYLVPWMREDLFARRDARKLKEVVIIEDDNPYDMEEMLMPLHKYLDAPTAHDIIHNGAT
FQIAGLTNNSHLSEAHEVRHCVQKFKSLGESVLQVAKRRLDSMLYVGLTEEHRESASLFA
NVVGSQVLSQVVPSNATAKIKALKSEASVTISETGSDKSNIQNGTSEVTLNKAEAKSGNM
TVKTLMEVYEGCITHLRKSQGTRRVNSLKRITPANFTRGTRTRVPKEVIQQIKSLNNLDV
ELYKYAKVIFAKEHELVSNKLISSSKRSIVDLPSELKSVLGEMGEEKLWKFVPVALMLLL
IVLFFLFVNAKRRRTSKVKI (SEQ ID No. 25)

A nucleic acid sequence encoding the plant, preferably Ara*bidopsis thaliana,* tyrosylprotein sulfotransferase may comprise SEQ ID No. 26, whereby nucleotide residues 1 to 72 of SEQ ID No. 26 represent a signal sequence and nucleotide residues 1350 to 1500, preferably 1380 to 1500, more preferably 1395 to 1500, more preferably 1407 to 1500, more preferably 1410 to 1500, in particular 1413 to 1500, of SEQ ID No. 25 represent the TS region (transmembrane domain with cytosolic tail; see e.g. Moore, PNAS 106(2009):14741-2):

ATGCAAATGAACTCTGTTTGGAAGCTGTCTCTTGGGTTATTACTTCTTAGCTCAGTTATT
GGCTCTTTTGCGGAACTTGATTTTGGCCATTGCGAAACTCTTGTGAAAAAATGGGCTGAT
TCTTCTTCATCTCGTGAAGAACATGTTAATAAAGACAAACGCTCGCTTAAGGATTTGCTC
TTCTTTCTCCACGTTCCGCGAACTGGAGGCAGAACATATTTTCATTGTTTTTGAGGAAG
TTGTATGATAGCTCTGAGGAATGTCCTCGATCTTACGACAAGCTCCACTTCAATCCAAGG
AAGGAAAAGTGCAAGTTGTTAGCCACACATGATGATTATAGTTTGATGGCAAAGCTTCCG
AGGGAGAGAACTTCGGTGATGACAATAGTTCGGGATCCTATTGCGCGTGTGTTAAGCACT
TATGAATTTTCCGTAGAGGTAGCAGCTAGGTTTTTGGTGCATCCCAATTTAACTTCTGCG
TCAAGGATGTCTAGCCGCATACGCAAGAGTAATGTAATAAGCACACTAGACATATGGCCA
TGGAAATACCTAGTTCCATGGATGAGAGAAGACTTGTTTGCTCGGCGAGATGCACGAAAA
TTGAAGGAGGTAGTGATCATTGAGGACGATAACCCGTATGACATGGAGGAGATGCTTATG
CCTTTGCACAAATATCTTGATGCGCCTACTGCTCATGACATCATCCACAATGGAGCGACT
TTTCAGATTGCAGGATTGACAAATAACTCCCATTTATCAGAAGCACACGAGGTTCGGCAT
TGTGTGCAGAAATTCAAAAGCCTTGGTGAGTCTGTTCTCCAAGTTGCCAAGAGGAGGCTA
GACAGCATGTTGTATGTTGGACTGACAGAGGAGCACAGGGAATCTGCATCACTTTTTGCC
AATGTAGTGGGTTCTCAAGTGCTGTCTCAAGTGGTTCCGTCCAATGCAACTGCGAAAATC
AAAGCTCTTAAATCAGAAGCAAGTGTCACAATTTCAGAAACCGGGTCAGATAAGAGTAAT
ATTCAGAATGGTACATCTGAAGTTACATTGAATAAGGCAGAAGCTAAGAGTGGGAATATG
ACGGTAAAAACCCTTATGGAAGTCTATGAAGGCTGCATCACTCATTTACGAAAGTCCCAA
GGAACCAGACGGGTCAACTCTCTGAAGAGAATAACTCCAGCAAATTTTACAAGAGGGACG

CGTACAAGAGTTCCTAAAGAGGTCATTCAGCAGATCAAATCGCTTAACAACCTCGATGTG
GAGCTCTACAAATATGCAAAAGTAATCTTTGCCAAAGAACATGAATTAGTGTCGAATAAG
TTGATCTCAAGTTCTAAGAGAAGCATTGTTGATCTGCCGAGTGAGTTAAAGAGCGTATTG
GGAGAAATGGGTGAAGAGAAGCTATGGAAGTTCGTACCAGTGGCATTGATGCTTTTATTG
ATCGTCCTCTTCTTTCTATTTGTAAACGCTAAAAGGAGAAGAACCTCCAAAGTTAAGATT
TGA (SEQ ID No. 26)

If a tyrosylprotein sulfotransferase comprising a TS region at its C-terminus (e.g. SEQ ID No. 25) is used according to the present invention, the TS region is preferably removed and the signal peptide located at the N-terminus is preferably exchanged with a heterologous CTS regions.

According to a preferred embodiment of the present invention the catalytically active fragment of a tyrosylprotein sulfotransferase comprises or consists of an amino acid sequence selected from the group consisting of amino acid residues 31 to 370, preferably 36 to 370, more preferably 38 to 370, more preferably 39 to 370, more preferably 41 to 370, more preferably 42 to 370, more preferably 43 to 370, more preferably 46 to 370, more preferably 51 to 370, in particular 40 to 370, of SEQ ID No. 1; amino acid residues 31 to 377, preferably 36 to 377, more preferably 39 to 377, more preferably 40 to 377, more preferably 42 to 377, more preferably 43 to 377, more preferably 46 to 377, more preferably 51 to 377, in particular 41 to 377, of SEQ ID No. 13; amino acid residues 31 to 370, preferably 36 to 370, more preferably 38 to 370, more preferably 39 to 370, more preferably 41 to 370, more preferably 42 to 370, more preferably 43 to 370, more preferably 46 to 370, more preferably 51 to 370, in particular 40 to 370, of SEQ ID No. 15; amino acid residues 41 to 390, preferably 46 to 390, more preferably 51 to 390, more preferably 53 to 390, more preferably 55 to 390, more preferably 56 to 390, more preferably 61 to 390, more preferably 66 to 390, in particular 54 to 390, of SEQ ID No. 16; amino acid residues 31 to 380, preferably 36 to 380, more preferably 38 to 380, more preferably 39 to 380, more preferably 41 to 380, more preferably 42 to 380, more preferably 43 to 380, more preferably 46 to 380, more preferably 51 to 380, in particular 40 to 380, of SEQ ID No. 19; amino acid residues 31 to 259, preferably 36 to 259, more preferably 39 to 259, more preferably 41 to 259, more preferably 43 to 259, more preferably 46 to 259, more preferably 51 to 259, of SEQ ID No. 20; amino acid residues 36 to 499, preferably 41 to 499, more preferably 46 to 499, more preferably 47 to 499, more preferably 49 to 499, more preferably 50 to 499, more preferably 51 to 499, more preferably 56 to 499, more preferably 61 to 499, in particular 48 to 499, of SEQ ID No. 23; and amino acid residues 449 to 500, preferably 25 to 459, more preferably 25 to 464, more preferably 25 to 468, more preferably 25 to 469, in particular 25 to 470, of SEQ ID No. 25.

According to a further preferred embodiment of the present invention the heterologous CTS region comprises a nucleic acid sequence selected from the group consisting of SEQ ID No. 3, SEQ ID No. 5 and SEQ ID No. 7.

The heterologous CTS region derived from a human tyrosylprotein sulfotransferase comprises preferably amino acid sequence SEQ ID No. 3:
MVGKLKQNLLLACLVISSVTVFYLGQHAMECHHRIEERS

SEQ ID No. 3 may be encoded by nucleic acid sequence SEQ ID No. 4:

atggttggaaagctgaagcagaacttactattggcatgtctggtgattagttctgtgact
gtgttttacctgggccagcatgccatggaatgccatcaccggatagaggaacgtagc

The heterologous CTS region derived from α1,3-Fucosyltransferase 11 of *Arabidopsis thaliana* comprises preferably amino acid sequence SEQ ID No. 5 (Met¹-Val⁶⁸ of AEE76217.1):

MGVFSNLRGPKIGLTHEELPVVANGSTSSSSPSSFKRKVSTFLPICVALVVIIEIGFLC
RLDNAS

SEQ ID No. 5 may be encoded by nucleic acid sequence SEQ ID No. 6:

atgggtgttttctccaatcttcgaggtcctaaaattggattgacccatgaagaattgcct
gtagtagccaatggctctacttcttcttcgtctccttcctctttcaagcgtaaagtc
tcgaccttttgccaatctgcgtggctcttgtcgtcattatcgagatcgggttcctctgt
cggctcgataacgcttct

The heterologous CTS region derived from α2,6-sialytransferase of *Rattus norvegicus* comprises preferably amino acid sequence SEQ ID No. 7 (Met¹-Gly⁵⁴ of M18769.1):
MIHTNLKKKFSLFILVFLLFAVICVWKKGSDYEALTLQAKEFQMPKSQEKVA

SEQ ID No. 7 may be encoded by nucleic acid sequence SEQ ID No. 8:

atgattcataccaacttgaagaaaaagttcagcctcttcatcctggtctttctcctgttc
gcagtcatctgtgtttggaagaaagggagcgactatgaggcccttacactgcaagccaag
gaattccagatgcccaagagccaggagaaagtggcc

The nucleic acid according to the present invention may encode a human tyrosylprotein sulfotransferase whose wild-type CTS region at its N-terminus has been replaced by a CTS region of α1,3-Fucosyltransferase 11 of *Arabidopsis thaliana* and may comprise or consist of SEQ ID No. 9:

atgggtgttttctccaatcttcgaggtcctaaaattggattgacccatgaagaattgcct
gtagtagccaatggctctacttcttcttcgtctccttcctctttcaagcgtaaagtc
tcgaccttttgccaatctgcgtggctcttgtcgtcattatcgagatcggttcctctgt
cggctcgataacgcttct(n)0-10 cagccagtcaaattggagagcacaaggaccactgt
gagaactggcctggacctcaaagccaacaaacctttgcctatcacaaagatatgccttt
aatatttattggaggtgtgcctcggagtggaaccacactcatgagggccatgctggacgc
acatcctgacattcgctgtggagaggaaaccagggtcattccccgaatcctggccctgaa
gcagatgtggtcacggtcaagtaaagagaagatccgcctggatgaggctggtgttactga
tgaagtgctggattctgccatgcaagccttcttactagaaattatcgttaagcatgggga
gccagccccttatttatgtaataaagatccttttgccctgaaatctttaacttacctttc
taggttattccccaatgccaaatttctcctgatggtccgagatggccggcatcagtaca
ttcaatgatttctcgaaaagttactatagctggatttgatctgaacagctatagggactg
tttgacaaagtggaatcgtgctatagagaccatgtataaccagtgtatggaggttggtta
taaaaagtgcatgttggttcactatgaacaacttgtcttacatcctgaacggtggatgag
aacactcttaaagttcctccagattccatggaaccactcagtattgcaccatgaagagat
gattgggaaagctggggggagtgtctctgtcaaaagtggagagatctacagaccaagtaat
caagccagtcaatgtaggagctctatcaaaatgggttggaagataccgccagatgtttt
acaagacatggcagtgattgctcctatgcttgccaagcttggatatgacccatatgccaa
cccacctaactacggaaaacctgatcccaaaattattgaaaacactcgaaggtctataa
gggagaattccaactacctgactttcttaaagaaaaaccacagactgagcaagtggagta
g

The nucleic acid molecule encoding a modified tyrosylprotein sulfotransferase comprising or consisting of SEQ ID No. 9 may comprise a linker between the nucleic acid stretch encoding a heterologous CTS region (italic) and the nucleic acid stretch encoding a tyrosylprotein sulfotransferase. This linker may consists of 1 to 10, preferably 2 to 9, more preferably 3 to 8, more preferably 4 to 7, in particular 6, nucleotides (n). This linker can be a result of fusing the heterologous CTS region to the tyrosylprotein sulfotransferase and thus comprise restriction sites or may have other functions. In a particularly preferred embodiment of the present invention the linker consists of or comprises the nucleic acid sequence GGATCC.

The nucleic acid according to the present invention may encode a human tyrosylprotein sulfotransferase whose wild-type CTS region at its N-terminus has been replaced by a CTS region of an α2,6-sialytransferase of *Rattus norvegicus* and may comprise or consist of SEQ ID No. 10:

atgattcataccaacttgaagaaaagttcagcctcttcatcctggtctttctcctgttc
gcagtcatctgtgtttggaagaaaggagcgactatgaggcccttacactgcaagccaag
gaattccagatgcccaagagccaggagaaagtggcc(n)0-10 cagccagtcaaattgga
gagcacaaggaccactgtgagaactggcctggacctcaaagccaacaaacctttgccta
tcacaaagatatgcctttaatatttattggaggtgtgcctcggagtggaaccacactcat
gagggccatgctggacgcacatcctgacattcgctgtggagaggaaaccaggtcattcc
ccgaatcctggccctgaagcagatgtggtcacggtcaagtaaagagaagatccgcctgga
tgaggctggtgttactgatgaagtgctggattctgccatgcaagccttcttactagaaat
tatcgttaagcatggggagccagccccttatttatgtaataaagatcctttttgccctgaa
atctttaacttacctttctaggttattccccaatgccaaatttctcctgatggtccgaga
tggccgggcatcagtacattcaatgatttctcgaaaagttactatagctggatttgatct
gaacagctatagggactgtttgacaaagtggaatcgtgctatagagaccatgtataacca
gtgtatggaggttggttataaaaagtgcatgttggttcactatgaacaacttgtcttaca
tcctgaacggtggatgagaacactcttaaagttcctccagattccatggaaccactcagt
attgcaccatgaagagatgattgggaaagctgggggagtgtctctgtcaaaagtggagag
atctacagaccaagtaatcaagccagtcaatgtaggagctctatcaaaatgggttggaa
gataccgccagatgttttacaagacatggcagtgattgctcctatgcttgccaagcttgg
atatgacccatatgccaacccacctaactacggaaaacctgatcccaaaattattgaaaa
cactcgaagggtctataaggagaattccaactacctgactttcttaaagaaaaccaca
gactgagcaagtggagtag

The nucleic acid molecule encoding a modified tyrosylprotein sulfotransferase comprising or consisting of SEQ ID No. 10 may comprise a linker between the nucleic acid stretch encoding a heterologous CTS region (italic) and the nucleic acid stretch encoding a tyrosylprotein sulfotransferase. This linker may consists of 1 to 10, preferably 2 to 9, more preferably 3 to 8, more preferably 4 to 7, in particular 6, nucleotides (n). This linker can be a result of fusing the heterologous CTS region to the tyrosylprotein sulfotransferase and thus comprise restriction sites or may have other functions. In a particularly preferred embodiment of the present invention the linker consists of or comprises the nucleic acid sequence CTCGAG.

Another aspect of the present invention relates to a polypeptide encoded by a nucleic acid molecule according to the present invention, preferably a polypeptide comprising or consisting of an amino acid sequence selected from the group consisting of SEQ ID No. 11 and 12.

The polypeptide according to the present invention may comprise or consist of a human tyrosylprotein sulfotransferase whose wild-type CTS region at its N-terminus has been replaced by a CTS region of an α1,3-Fucosyltransferase 11 of *Arabidopsis thaliana* and may comprise or consist of SEQ ID No. 11:

MGVFSNLRGPKIGLTHEELPVVANGSTSSSSSPSSFKRKVSTFLPICVALVVIIEIGFLC
RLDNAS(X)1-5 QPVKLESTRTTVRTGLDLKANKTFAYHKDMPLIFIGGVPRSGTTLMRA
MLDAHPDIRCGEETRVIPRILALKQMWSRSSKEKIRLDEAGVTDEVLDSAMQAFLLEIIV
KHGEPAPYLCNKDPFALKSLTYLSRLFPNAKFLLMVRDGRASVHSMISRKVTIAGFDLNS
YRDCLTKWNRAIETMYNQCMEVGYKKCMLVHYEQLVLHPERWMRTLLKFLQIPWNHSVLH
HEEMIGKAGGVSLSKVERSTDQVIKPVNVGALSKWVGKIPPDVLQDMAVIAPMLAKLGYD
PYANPPNYGKPDPKIIENTRRVYKGEFQLPDFLKEKPQTEQVE

The modified tyrosylprotein sulfotransferase comprising or consisting of SEQ ID No. 11 may comprise a linker between the heterologous CTS region (italic) and the tyrosylprotein sulfotransferase. This linker may consists of 1 to 5, preferably 1 to 4, more preferably 2 to 5, more preferably 2 or 3, in particular 2, amino acid residues (X). This linker can be a result of fusing the heterologous CTS region to the tyrosylprotein sulfotransferase or may have other functions. In a particularly preferred embodiment of the present invention the linker consists of or comprises the amino acid sequence GS.

The polypeptide according to the present invention may comprise or consist of a human tyrosylprotein sulfotransferase whose wild-type CTS region at its N-terminus has been replaced by a CTS region of an α2,6-sialytransferase of *Rattus norvegicus* and may comprise or consist of SEQ ID No. 12:

MIHTNLKKKFSLFILVFLLFAVICVWKKGSDYEALTLQAKEFQMPKSQEKVA(X)1-5
QPVKLESTRTTVRTGLDLKANKTFAYHKDMPLIFIGGVPRSGTTLMRAMLDAHPDIRCGE
ETRVIPRILALKQMWSRSSKEKIRLDEAGVTDEVLDSAMQAFLLEIIVKHGEPAPYLCNK
DPFALKSLTYLSRLFPNAKFLLMVRDGRASVHSMISRKVTIAGFDLNSYRDCLTKWNRAI
ETMYNQCMEVGYKKCMLVHYEQLVLHPERWMRTLLKFLQIPWNHSVLHHEEMIGKAGGVS
LSKVERSTDQVIKPVNVGALSKWVGKIPPDVLQDMAVIAPMLAKLGYDPYANPPNYGKPD
PKIIENTRRVYKGEFQLPDFLKEKPQTEQVE

The modified tyrosylprotein sulfotransferase comprising or consisting of SEQ ID No. 10 may comprise a linker between the heterologous CTS region (italic) and the tyrosylprotein sulfotransferase. This linker may consists of 1 to 5, preferably 1 to 4, more preferably 2 to 5, more preferably 2 or 3, in particular 2, amino acid residues (X). This linker can be a result of fusing the heterologous CTS region to the tyrosylprotein sulfotransferase or may have other functions. In a particularly preferred embodiment of the present invention the linker consists of or comprises the amino acid sequence LE.

The term "polypeptide" is used herein interchangeably with the term "protein" and both terms refer to a polymer of amino acid residues.

Another aspect of the present invention relates to a vector comprising a nucleic acid molecule according to the present invention.

The vector of the present invention can be used to clone the nucleic acid molecule of the present invention, as a shuttle or as an expression vector in host cells. Expression vectors may include an expression cassette which comprises various specified nucleic acid elements which permit transcription of a particular nucleic acid in a host cell. Typically, expression cassettes comprise, among other sequences, a nucleic acid to be transcribed, a promoter and a terminator.

The vector of the present invention can be designed to be integrated into the genome of the host cell. Alternatively the vector is designed to not integrate into the genome of a host cell allowing transient expression of the nucleic acid molecule of the present invention. In the latter case the vector remains in a non-integrated state free within the cell.

A "vector" according to the present invention refers to a nucleic acid used to introduce the nucleic acid molecule of the present invention into a host cell. Expression vectors permit transcription of a nucleic acid inserted therein.

In order to enable a host cell to express polypeptide of the present invention encoded by the nucleic acid molecule as defined above the vector of the present invention comprises a promoter operably linked to the nucleic acid molecule.

As used herein, "operably linked" refers to a functional linkage between a promoter and the nucleic acid molecule encoding the polypeptide of the present invention, wherein the promoter sequence initiates and mediates transcription of the DNA sequence corresponding to said nucleic acid molecule.

The term "promoter", as used herein, refers to a region of a nucleic acid molecule upstream from the start of transcription and involved in recognition and binding of RNA polymerase and other proteins to initiate transcription. Promoters are able to control (initiate) transcription in a cell. Plant promoters are able of initiating transcription in plant cells whether or not its origin is a plant cell. Such promoters include promoters obtained from plants, plant viruses and bacteria which comprise genes expressed in plant cells such Agrobacterium or Rhizobium. The promoter used in the vector of the present invention can be "inducible" or "repressible", i.e. under environmental control. Such promoters can be controlled by changing the cultivation conditions (e.g. temperature) or by adding specific substances. Of course, the promoter used in the vectors of the present invention may be a "constitutive" promoter. Constitutive promoters are active under most environmental conditions and express continuously a protein or polypeptide of interest.

According to a preferred embodiment of the present invention the promoter is selected from the group consisting of promoters active in plants and plant cells, like the cauliflower mosaic virus 35S promoter, opine (octopine, nopaline, etc.) synthase promoters, actin promoter, ubiquitine promoter, etc.

In order to prevent transcriptional activation of downstream nucleic acid sequences by upstream promoters the vector of the present invention may comprise a "terminator" or "terminator sequence". According to a preferred embodiment of the present invention the vector comprises a terminator which is preferably a g7T terminator.

Another aspect of the present invention relates to a host cell comprising a nucleic acid molecule or a vector according to the present invention.

A "host cell", as used herein, refers to a cell that contains a nucleic acid molecule or a vector and supports the replication and/or expression of said nucleic acid molecule or said vector. Host cells may be prokaryotic cells such as *E. coli* or eukaryotic cells such as yeast, plant, insect, amphibian or mammalian cells. In a particular preferred embodiment of the present invention the host cell is a plant cell. For cloning purposes and/or for producing a nucleic acid molecule of the present invention it is preferred to use prokaryotic cells, in particular *E. coli.* It is particularly preferred to use plant cells as host cells.

Another aspect of the present invention relates to a plant or plant cell capable of transferring a sulfate moiety to a tyrosine residue of a polypeptide which is a substrate of a tyrosylprotein sulfotransferase and preferably of animal origin heterologously produced in said plant or plant cell comprising a nucleic acid molecule or a vector according to the present invention.

A "substrate of a tyrosylprotein sulfotransferase" is any polypeptide or protein which can be sulfated in the presence of a tyrosylprotein sulfotransferase and a sulfate donor like phosphoadenosine-5'-phosphosulfate. Such substrates can be identified, for instance, by contacting and incubating them with a tyrosylprotein sulfotransferase and a sulfate donor (see e.g. Seibert C et al (Biochemistry 47(2008): 11251-11262)). Particularly preferred substrates are polypeptides and proteins of animal origin. Exemplary polypeptides which can be used as substrate for a tyrosylprotein sulfotransferase include PG9, PG16, hirudin and gp120.

"Polypeptides of animal origin", as used herein, refers to polypeptides which are not naturally occurring in plants and plant cells or any other non-animal organism. Polypeptides of animal origin can be derived from the genome of animals and are usually expressed in animals. However, polypeptides derived from non-animal organisms which are homologs of animal derived polypeptides are not encompassed by this definition.

The term "plant cell", as used herein, refers to protoplasts, gamete producing cells and cells which regenerate into whole plants. Plant cells, as used herein, further include cells obtained from or found in seeds, suspension cultures, embryos, meristematic regions, callus tissue, leaves, roots, shoots, gametophytes, sporophytes, pollen and microspores.

The plant and plant cell of the present invention may carry the nucleic acid molecule of the present invention in a non-integrated form (e.g. as a vector) or may be a "transgenic plant" or "transgenic plant cell". Such a plant or plant cell comprises within its genome a heterologous nucleic acid molecule. This heterologous nucleic acid molecule is usually stably integrated within the genome such that the polynucleotide is passed on to successive generations. In order to allow the plant and plant cell to express the polypeptide of the present invention, its encoding nucleic acid molecule is operably linked to a promoter.

The nucleic acid molecule and the vector according to the present invention enable a plant or plant cell to sulfate polypeptides and proteins expressed in said plant or plant cell. Therefore, the transgenic plant or plant cell of the present invention comprise preferably a nucleic acid molecule encoding a heterologous polypeptide operably linked to a promoter region.

A "heterologous protein" or "heterologous polypeptide", as defined herein, refers to a protein or polypeptide that is not expressed by the plant or plant cell in nature. This is in contrast with a homologous protein which is a protein naturally expressed by a plant or plant cell. The heterologous expression of polypeptides and proteins within a host cell can be achieved by means and methods known in the art and described above for the polypeptide of the present invention.

Exemplary plants to be used according to the present invention include Nicotiana spp, Arabidopsis thaliana, Algae, duck-weed (*Lemna minor*)*,* mosses (e.g. *Physcomitrella*)*,* corn (*Zea mays*)*,* rice (*Oryza sativa*)*,* wheat (*Triticum*)*,* peas (*Pisum sativum),* flax (*Linum usitatissimum*) and rapeseed (*Brassica napus*)*.*

According to a preferred embodiment of the present invention the heterologous polypeptide of animal origin is a mammalian, more preferably human, polypeptide.

According to a further embodiment of the present invention the heterologous animal polypeptide is an antibody.

Examples of heterologous polypeptides and proteins that can be advantageously produced in a sulfated form by the plants or plant cells of the present invention include antibodies or fragments thereof which are selected from the group consisting of monoclonal antibodies, chimeric antibodies, humanized antibodies, human antibodies, multispecific antibodies, Fab, Fab', F(ab')2, Fv, domain antibody (dAb), complementarity determining region (CDR) fragments, CDR-grafted antibodies, single-chain antibodies (ScFv), single chain antibody fragments, chimeric antibodies, diabodies, triabodies, tetrabodies, minibodies, linear antibodies, chelating recombinant antibodies, tribodies, bibodies, intrabodies, nanobodies, small modular immunopharmaceuticals (SMIP), camelized antibodies, VHH containing antibodies and polypeptides that comprise at least a portion of an immunoglobulin that is sufficient to confer specific antigen binding to the polypeptide, such as one, two, three, four, five or six CDR sequences.

According to a particularly preferred embodiment of the present invention the antibody is an antibody binding to an HIV surface protein.

As used herein, the term "specific for" can be used interchangeably with "binding to" or "binding specifically to". These terms characterize molecules that bind to an antigen or a group of antigens with greater affinity (as determined by, e.g., ELISA or BIAcore assays) than other antigens or groups of antigens. According to the present invention molecules "specific for" an antigen may also be able to bind to more than one, preferably more than two, more preferably more than three, even more preferably more than five, antigens. Such molecules are defined to be "cross-reactive" (e.g. cross-reactive immunoglobulins, cross-reactive antigen binding sites).

The antibody expressed and sulfated by the plant cell and plant of the present invention is preferably an antibody selected from the group consisting of PG9, PG16, **47e,** 412d, Sb1, C12, E51, CM51 and a variant thereof.

It is known that sulfated antibodies may have a greater binding affinity to an antigen compared to its non-sulfated version. This is effect is particularly significant for antibodies like PG9, PG16 (see e.g. Pejchal R at al. Proc Natl Acad Sci USA 107(2010):11483-11488),PGT141-145 (see e.g. McLellan JS et al. Nature 480(2011):336-343), 47e, 412d, Sb1, C12, E51, CM51 (see e.g. Choe H et al. Cell 114(2003):161-170 and Huang CC et al. Proc Natl Acad Sci USA 101(2004):2706-2711) and variants thereof. Therefore, it is particularly advantageous to provide efficient tools for producing sulfated PG9, PG16, PGT141-145, 47e, 412d, Sb1, C12, E51, CM51 and variants thereof.

According to a preferred embodiment of the present invention the antibody variant is selected from the group consisting of PG9 comprising modifications at R^{L94}SH^{L95A}.

According to a further embodiment of the present invention the plant is *Nicotiana benthamiana, Nicotiana spp, Arabidopsis thaliana, Algae, Lemna minor, Physcomitrella, Zea mays, Oryza sativa, Triticum, Pisum sativum, Linum usitatissimum* or *Brassica napus,* whereby *Nicotiana benthamiana* is particularly preferred.

Another aspect of the present invention relates to a method of recombinantly producing a polypeptide of animal origin carrying an animal-type sulfation comprising the step of cultivating a plant or plant cell according to the present invention.

Methods and means to cultivate recombinant plants and plant cells are known in the art.

The present invention is further illustrated by the following figures and examples without being restricted thereto.

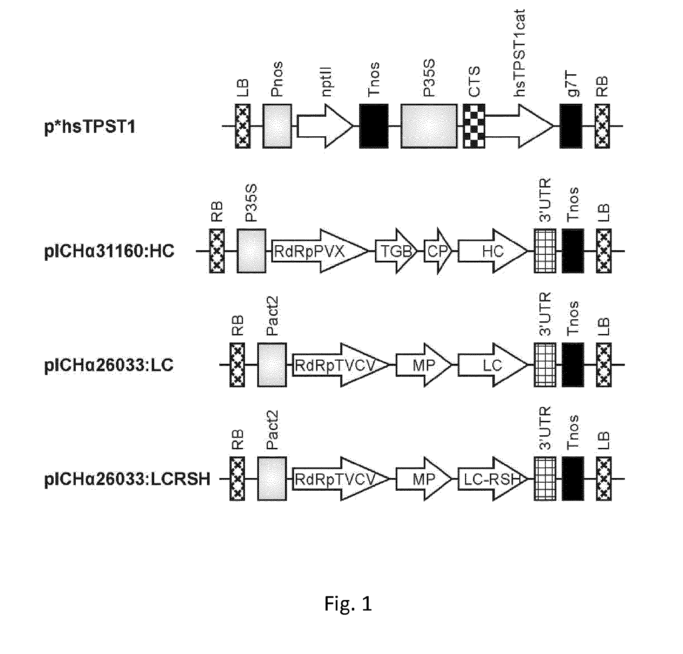

Fig. 1 shows the PG9 and TPST1 constructs used in the examples. PG9HC, PG9LC and PG9LC-RSH were cloned into MagnIcon vectors. Three versions of hsTPST1 containing different CTS regions were cloned into pPT2 giving rise to p^{Fulll}hsTPST1, p^{RST}hsTPST1 and p^{Fut11}hsTPST1.

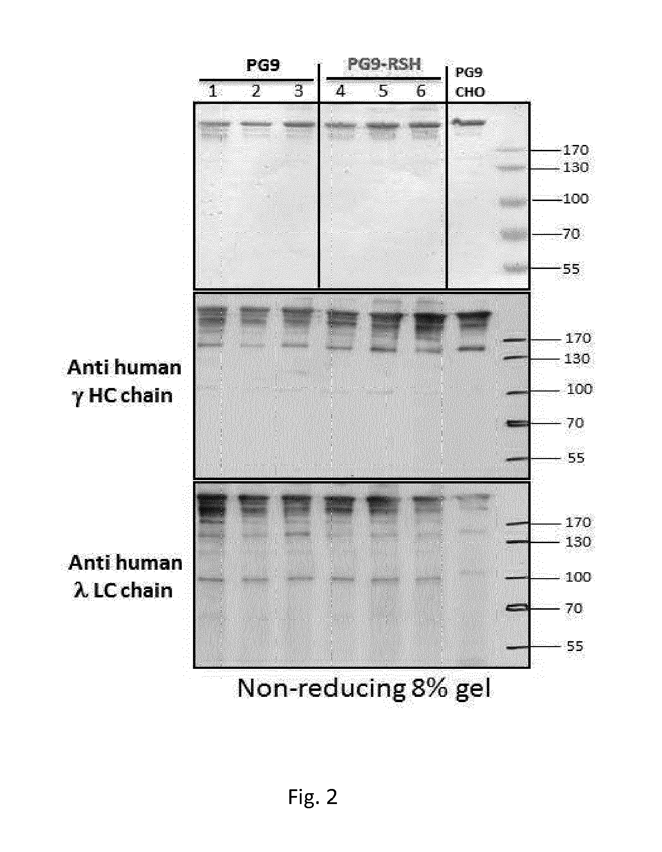

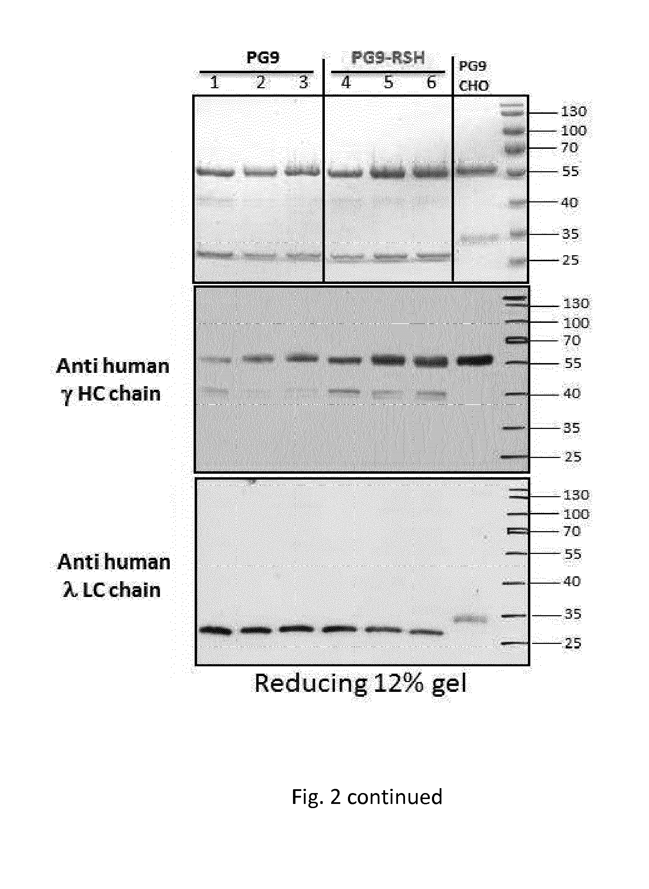

Fig. 2 shows the purification of plant-produced PG9 and RSH and comparison to ^{CHO}PG9. Coomassie staining (top panels) and immunoblotting (middle and bottom panels) of purified PG9 and RSH after separation by SDS-PAGE under reducing (right) and non-reducing (left) conditions. 1: ^{ΔXF}PG9; 2: ^{ΔXF}PG9_{Sulf}; 3: ^{ΔXF}PG9_{SulfSia}; 4: ^{ΔXF}RSH; 5: ^{ΔXF}RSH_{Sulf}; 6: ^{ΔXF}RSH_{SulfSia}.

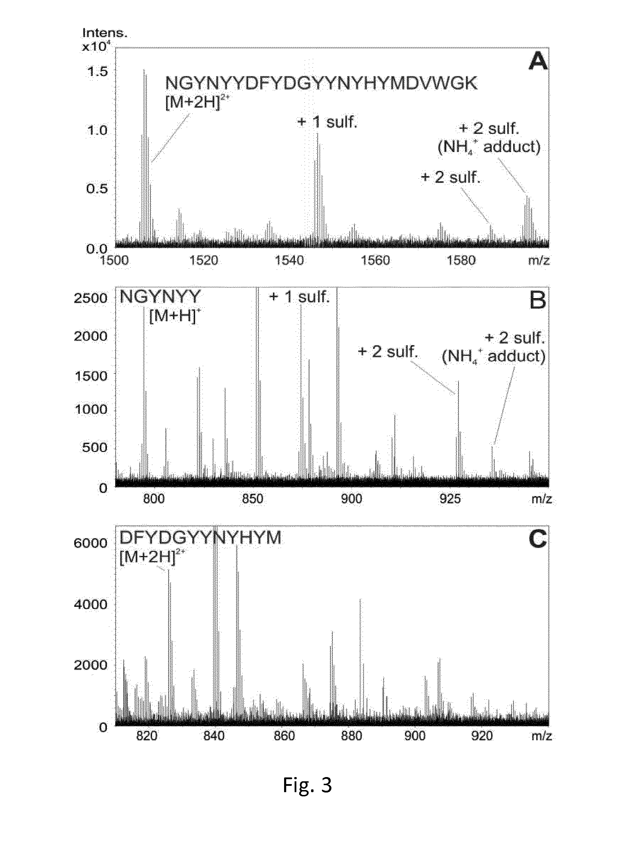

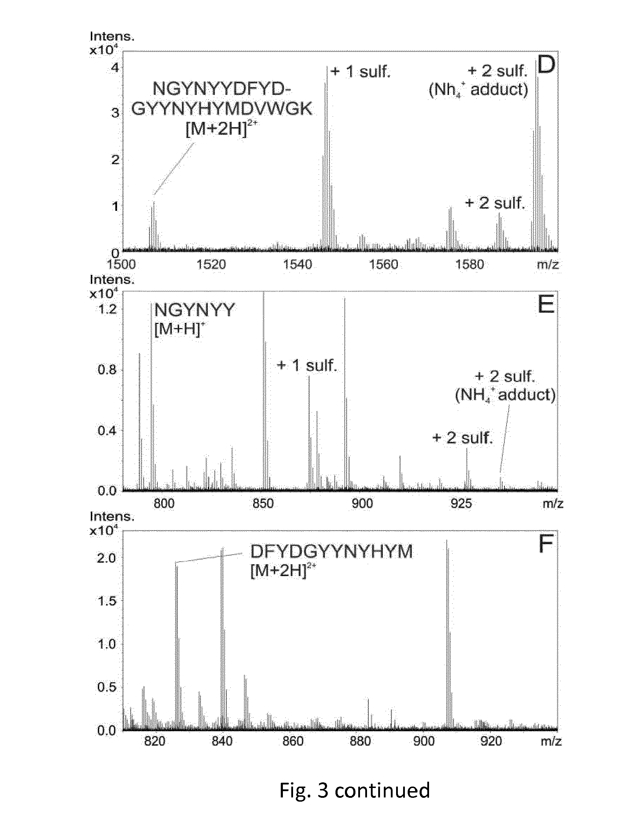

Fig. 3 shows ^{ΔXF}PG9 and ^{CHO}PG9 are singly and doubly sulfated on tyrosine residues Y^{100E}, Y^{100G} or Y^{100H}. The sulfation sites of ^{ΔXF}PG9 and ^{CHO}PG9 were mapped by LC-ESI-MS to the tryptic PG9 peptide N^{100C}GYNYYDFYDGYYNYHYMDVWGK¹⁰⁵ (SEQ ID No. 27) (panels A and D, respectively). Further digestion with AspN revealed non-sulfated, singly and doubly sulfated variants of the peptide N^{100C}GYNYY^{100H} (SEQ ID No. 28) (panels B and E for ^{ΔXF}PG9 and

^{CHO}PG9). No sulfated residues were found on the other AspN fragment, D^{100I}FYDGYYNYHYM^{100T} (SEQ ID No. 29) (panels C and F for ^{ΔXF}PG9 and ^{CHO}PG9).

### EXAMPLES:

### MATERIALS AND METHODS:

### 1. Cloning of neutralizing anti-HIV antibody PG9 and its variant RSH

The signal peptide of barley α-amylase (amino acid residues 1 to 24 of the amino acid sequence of acc. no. CAX51374.1) was cloned into MagnIcon vectors pICH26033 and pICH31160 (Niemer, M., et al. Biotechn J 9(2014):493-500) to give rise to pICHα26033 and pICHα31160. cDNA codon-optimized for *Nicotiana benthamiana* and encoding a BsaI site followed by the PG9 (McLellan JS et al. Nature 480(2011):336-343; Protein Data Bank accession nos. 3U36_A, 3U36_B, 3U36_C, 3U36_D, 3U36_E, 3U36_F) variable heavy and C_{H}1 domain without signal peptide was PCR-amplified.

PG9LC (SEQ ID No. 30; PDB-database: 3U2S, 3U36, 3U4E, 3MUH) and PG9LC-RSH (SEQ ID No. 32) cDNAs (both encoding an N23Q mutation) were synthesized with codons optimized for *Nicotiana benthamiana,* without signal peptide but with 5'- and 3' BsaI restriction sites.

PG9LC consists of amino acid sequence SEQ ID No. 30, whereby the signal peptide of barley α-amylase is marked in bold and italic:

MANKHLSLSLFLVLLGLSASLASGQSALTQPASVSGSPGQSITISCQGTSNDVGGYESVS
WYQQHPGKAPKVVIYDVSKRPSGVSNRFSGSKSGNTASLTISGLQAEDEGDYYCKSLTST
RRRVFGTGTKLTVLGQPKAAPSVTLFPPSSEELQANKATLVCLISDFYPGAVTVAWKADS
SPVKAGVETTTPSKQSNNKYAASSYLSLTPEQWKSHKSYSCQVTHEGSTVEKTVAPTECS

PG9LC is encoded by nucleotide sequence SEQ ID No. 31 (including stop codon), whereby the nucleic acid stretch encoding the signal peptide of barley α-amylase is marked in bold and italic:

ATGGCGAACAAACACTTGTCCCTCTCCCTCTTCCTCGTCCTCCTTGGCCTGTCGGCCAGC
TTGGCCTCAGGTCAGAGTGCTCTTACTCAGCCTGCTTCTGTTTCTGGTTCTCCTGGTCAG
AGCATCACCATTTCTTGCCAGGGAACCTCTAACGATGTGGGAGGTTACGAGTCCGTGTCT
TGGTATCAACAGCATCCTGGTAAGGCTCCTAAGGTGGTGATCTACGATGTGAGCAAGAGG

CCTTCTGGTGTGAGCAATAGGTTCAGCGGTAGCAAGTCTGGTAACACCGCTTCTCTTACC
ATCTCTGGACTTCAGGCTGAGGATGAGGGAGATTACTACTGCAAGTCTCTGACCTCCACT
AGAAGAAGGGTGTTCGGAACCGGTACTAAGCTTACTGTTCTGGGTCAACCTAAGGCTGCT
CCTTCTGTGACTTTGTTCCCTCCATCTTCTGAGGAACTGCAGGCTAACAAGGCTACCCTT
GTGTGCCTGATCAGCGATTTTTACCCTGGTGCTGTTACCGTGGCTTGGAAGGCTGATTCT
TCACCTGTTAAGGCTGGTGTGGAAACCACCACTCCTAGCAAGCAGAGCAACAACAAGTAC
GCTGCTAGCTCCTACCTTAGCCTTACTCCTGAACAGTGGAAGTCCCACAAGAGCTACTCA
TGCCAGGTTACCCATGAGGGTTCTACCGTGGAAAAGACTGTTGCTCCTACTGAGTGCAGC
TAG

PG9LC-RSH consists of amino acid sequence SEQ ID No. 32, whereby the signal peptide of barley α-amylase is marked in bold and italic:

MANKHLSLSLFLVLLGLSASLASGQSALTQPASVSGSPGQSITISCQGTSNDVGGYESVS
WYQQHPGKAPKVVIYDVSKRPSGVSNRFSGSKSGNTASLTISGLQAEDEGDYYCKSLTSR
SHRVFGTGTKLTVLGQPKAAPSVTLFPPSSEELQANKATLVCLISDFYPGAVTVAWKADS
SPVKAGVETTTPSKQSNNKYAASSYLSLTPEQWKSHKSYSCQVTHEGSTVEKTVAPTECS

PG9LC-RSH is encoded by nucleotide sequence SEQ ID No. 33 (including stop codon), whereby the nucleic acid stretch encoding the signal peptide of barley α-amylase is marked in bold and italic:

ATGGCGAACAAACACTTGTCCCTCTCCCTCTTCCTCGTCCTCCTTGGCCTGTCGGCCAGC
TTGGCCTCAGGTCAGAGTGCTCTTACTCAGCCTGCTTCTGTTTCTGGTTCTCCTGGTCAG
AGCATCACCATTTCTTGCCAGGGAACCTCTAACGATGTGGAGGTTACGAGTCCGTGTCT
TGGTATCAACAGCATCCTGGTAAGGCTCCTAAGGTGGTGATCTACGATGTGAGCAAGAGG
CCTTCTGGTGTGAGCAATAGGTTCAGCGGTAGCAAGTCTGGTAACACCGCTTCTCTTACC
ATCTCTGGACTTCAGGCTGAGGATGAGGGAGATTACTACTGCAAGTCTCTGACCTCCAGA
AGTCACAGGGTGTTCGGAACCGGTACTAAGCTTACTGTTCTGGGTCAACCTAAGGCTGCT
CCTTCTGTGACTTTGTTCCCTCCATCTTCTGAGGAACTGCAGGCTAACAAGGCTACCCTT
GTGTGCCTGATCAGCGATTTTTACCCTGGTGCTGTTACCGTGGCTTGGAAGGCTGATTCT
TCACCTGTTAAGGCTGGTGTGGAAACCACCACTCCTAGCAAGCAGAGCAACAACAAGTAC
GCTGCTAGCTCCTACCTTAGCCTTACTCCTGAACAGTGGAAGTCCCACAAGAGCTACTCA
TGCCAGGTTACCCATGAGGGTTCTACCGTGGAAAAGACTGTTGCTCCTACTGAGTGCAGC
TAG

PG9LC and PG9LC-RSH were inserted into pICHa26033 in frame after the barley α-amylase signal peptide. All vectors were transformed into *E. coli* by electroporation and upon sequence confirmation into the *Agrobacterium tumefaciens* strain GV3101pMP90.

### 2. Cloning of tyrosylprotein sulfotransferase (TPST) constructs

For expression in plants, hsTPST1 (accession number AK313098.1, open reading frame from start to stop codon) was cloned into vector pPT2 (Strasser R et al. Biochem J 387(2005):385-391). Three different constructs containing different CTS regions were constructed (Fig. 1). p^{Full}hsTPST1 contains the authentic CTS region (SEQ ID No. 3), in p^{Fut11}hsTPST1 Met¹-Ser³⁹ of hsTPST1 is replaced by the CTS of A. *thaliana* Fut11 (Met¹-Val⁶⁸) (SEQ ID No. 5), and in p^{RST}hsTPST it is replaced by the CTS region of rat sialyltransferase (Met¹-Gly⁵⁴) (SEQ ID No. 7). The nucleic acid sequences encoding the respective polypeptides having SEQ ID No. 1, 11 and 12 consist of SEQ ID No. 2, 9 and 10, respectively. After transformation into *E. coli* and sequence confirmation, all constructs were transformed into *Agrobacterium tumefaciens* strain UIA143pMP90.

### 3. In planta expression of PG9 and RSH

*N. benthamiana* ΔXT/FT plants (age: 4-5 weeks) were used for co-infiltration with agrobacteria as described previously (Strasser R, et al. Plant Biotech J 6(2008):392-402). Briefly, liquid cultures of agrobacterial strains carrying pPG9HC, pPG9LC, pPG9LC-RSH, p^{Full}hsTPST1, P^{Fut11}hsTPST1 and p^{RST}hsTPST1 were grown overnight, pelleted and resuspended in infiltration buffer (25 mM MES (pH 5.5), 25 mM MgSO₄, 0.1 mM acetosyringone). Mixtures of bacteria containing pPG9HC and pPG9LC (or pPG9LC-RSH) were infiltrated with or without different TPST constructs into *N. benthamiana* ΔXT/FT leaves. For *in planta* galactosylation and sialylation, an additional 6 genes had to be infiltrated (Castilho A et al. J Biol Chem 285(2010):15923-15930). OD₆₀₀ for infiltration was 0.01 for each of the IgG vectors, up to 0.8 for the TPST constructs and 0.05 for the vectors required for galactosylation/sialylation. Plants were harvested 3-6 days post infiltration.

### 4. Cloning, expression and purification of gp120^{ZM109}

The codon-optimized coding sequence (SEQ ID No. 34) for gp120 of HIV strain ZM109 (gp120^{ZM109}) (SEQ ID No. 35) was appended with a C-terminal hexahistidine tag (bold and underlined in SEQ ID No. 34 and 35) and inserted into the HindIII/NotI sites of pCEP4 (Life Technologies).

gp120^{ZM109} (including a C-terminal histidine tag) is encoded by the nucleic acid sequence SEQ ID No. 34:

ATGCCTATGGGCAGCCTGCAGCCCCTGGCCACACTGTATCTGCTGGGAATGCTGGTGGCC
AGCTGCCTGGGCGTGTGGAAAGAGGCCAAGACCACCCTGTTCTGCGCCAGCGACGCCAAG
AGCTACGAGCGCGAGGTGCACAATGTGTGGGCCACCCATGCCTGCGTGCCCACCGATCCT
GATCCCCAGGAACTCGTGATGGCCAACGTGACCGAGAACTTCAACATGTGGAAGAACGAC
ATGGTGGACCAGATGCACGAGGACATCATCAGCCTGTGGGACCAGAGCCTGAAGCCCTGC
GTGAAGCTGACCCCTCTGTGCGTGACCCTGAACTGCACATCTCCTGCCGCCCACAACGAG
AGCGAGACAAGAGTGAAGCACTGCAGCTTCAACATCACCACCGACGTGAAGGACCGGAAG
CAGAAAGTGAACGCCACCTTCTACGACCTGGACATCGTGCCCCTGAGCAGCAGCGACAAC
AGCAGCAACAGCTCCCTGTACAGACTGATCAGCTGCAACACCAGCACCATCACCCAGGCC
TGCCCCAAGGTGTCCTTCGACCCCATCCCCATCCACTACTGTGCCCCTGCCGGCTACGCC
ATCCTGAAGTGCAACAACAAGACCTTCAGCGGCAAGGGCCCCTGCAGCAACGTGTCCACC
GTGCAGTGTACCCACGGCATCAGACCCGTGGTGTCCACCCAGCTGCTGCTGAATGGCAGC
CTGGCCGAAGAGGAAATCGTGATCAGAAGCGAGAACCTGACCGACAACGCCAAGACAATC
ATTGTGCATCTGAACAAGAGCGTGGAAATCGAGTGCATCAGGCCCGGCAACAACACCAGA
AAGAGCATCAGACTGGGCCCTGGCCAGACCTTTTACGCCACCGGGGATGTGATCGGCGAC
ATCCGGAAGGCCTACTGCAAGATCAACGGCAGCGAGTGGAACGAGACACTGACAAAGGTG
TCCGAGAAGCTGAAAGAGTACTTTAACAAGACCATTCGCTTCGCCCAGCACTCTGGCGGC
GACCTGGAAGTGACCACCCACAGCTTCAATTGCAGAGGCGAGTTCTTCTACTGCAATACC
AGCGAGCTGTTCAACAGCAACGCCACCGAGAGCAATATCACCCTGCCCTGCCGGATCAAG
CAGATCATCAATATGTGGCAGGGCGTGGGCAGAGCTATGTACGCCCCTCCCATCCGGGGC
GAGATCAAGTGCACCTCTAACATCACCGGCCTGCTGCTGACCAGGGACGGCGGAAACAAC
AACAATAGCACCGAGGAAATCTTCCGGCCCGAGGGCGGCAACATGAGAGACAATTGGAGA
TCCGAGCTGTACAAGTACAAGGTGGTGGAAATCAAGGGCCTGCGGGGCAGCCACCACCAT
CATCACCATTGA

gp120^{ZM109} (including a C-terminal histidine tag) consists of the amino acid sequence SEQ ID No. 35:

MPMGSLQPLATLYLLGMLVASCLGVWKEAKTTLFCASDAKSYEREVHNVWATHACVPTDP
DPQELVMANVTENFNMWKNDMVDQMHEDIISLWDQSLKPCVKLTPLCVTLNCTSPAAHNE
SETRVKHCSFNITTDVKDRKQKVNATFYDLDIVPLSSSDNSSNSSLYRLISCNTSTITQA
CPKVSFDPIPIHYCAPAGYAILKCNNKTFSGKGPCSNVSTVQCTHGIRPVVSTQLLLNGS
LAEEEIVIRSENLTDNAKTIIVHLNKSVEIECIRPGNNTRKSIRLGPGQTFYATGDVIGD
IRKAYCKINGSEWNETLTKVSEKLKEYFNKTIRFAQHSGGDLEVTTHSFNCRGEFFYCNT
SELFNSNATESNITLPCRIKQIINMWQGVGRAMYAPPIRGEIKCTSNITGLLLTRDGGNN
NNSTEEIFRPEGGNMRDNWRSELYKYKVVEIKGLRGSHHHHHH

Transient expression in FreeStyle™293F cells (Life Technologies) was performed following the instructions of the manufacturer. Culture supernatants were subjected to affinity chromatography using Ni²⁺-charged Chelating Sepharose (GE Healthcare), omitting the addition of phosphatase and protease inhibitors. Fractions eluted with 250 mM imidazole were dialyzed against PBS containing 0.02% (v/v) NaN₃ and then concentrated by ultrafiltration.

### 5. Monoclonal antibody (mAb) purification

Leaf material (see item 3.) infiltrated with the transformed *Agrobacterium tumefaciens* strains described under item 2 was crushed under liquid nitrogen, extracted twice for 20 min on ice with 45 mM Tris/HCl (pH 7.4) containing 1.5 M NaCl, 40 mM ascorbic acid and 1 mM EDTA (2 ml per g leaf material) and cleared by centrifugation (4°C, 30 min, 27.500 g). Upon vacuum filtration through 10-µm cellulose filters (Roth, AP27.1) and centrifugation (4°C, 30 min, 27.500 g), the extract was filtered through a series of filters with pore sizes ranging from 10 µm to 0.2 µm (Roth, AP27.1, Roth, AP51.1, Roth, CT92.1, Roth, KH54.1) before being applied to a 1.5 ml Protein A Sepharose 4FF column (GE Healthcare, 17-1279-01) at 1 ml/min. Upon washing with PBS, bound mAbs were eluted with 100 mM glycine/HCl (pH 2.5). The eluate was immediately neutralized (1 M Tris/HCl (pH 8.0), 1.5 M NaCl), mAb-containing fractions were identified by their absorbance at 280 nm, pooled and the buffer was exchanged to PBS by dialysis.

### 6. SDS-PAGE and western blotting

Samples were separated on 12% polyacrylamide gels under reducing conditions and on 8% polyacrylamide gels under non-reducing conditions, followed by either staining with Coomassie Brilliant Blue or blotting onto a nitrocellulose membrane (GE Healthcare). mAb heavy and light chains were detected with anti-human IgG gamma chain-peroxidase conjugate (Sigma A8775) or anti-human lambda light chain-peroxidase conjugate (Sigma A5175) and visualized with a chemiluminescence detection kit (Bio-Rad).

### 7. Glycosylation analysis of mAbs and gp120

The *N*-glycosylation profiles of mAbs (Asn²⁹⁷) and gp120^{ZM109} (Asn¹⁶⁰, Asn¹⁷³) were determined by LC-ESI-MS as published by Stadlmann et al. (Proteomics 8(2008):2858-2871) and Pabst et al. (Biol Chem 393(2012):719-730), respectively. Briefly, purified mAbs or gp120 were separated by reducing SDS-PAGE, stained with Coomassie Brilliant Blue and the relevant bands were excised from the gel. Upon S-alkylation with iodoacetamide and tryptic or tryptic/chymotryptic digestion (gp120^{ZM109} Asn¹⁷³), fragments were eluted from the gel with 50% acetonitrile and separated on a reversed phase column (150 x 0.32 mm BioBasic-18, Thermo Scientific) with a gradient of 1-80% acetonitrile. Glycopeptides were analysed with a Q-TOF Ultima Global mass spectrometer (Waters). Spectra were summed and deconvoluted for identification of glycoforms. Annotation of glycoforms was done according to the proglycan nomenclature (Stadlmann J. et al. (Proteomics 8(2008), 2858-2871).

### 8. Sulfation analysis of PG9 and RSH

Tryptic peptides were prepared as above (see item 7), digested with AspN where appropriate and then separated using a Dionex Ultimate 3000 HPLC system using a Thermo BioBasic C18 separation column (5 µm particle size, 150 x 0.32 mm) with a gradient from 95% solvent A (65 mM ammonium formate) and 5% solvent B (acetonitrile) to 75% B in 50 min at a flow rate of 6 µL/min. Peptides were analysed on a maXis 4G ETD QTOF mass spectrometer (Bruker Daltonik) equipped with the standard ESI source in the positive ion, DDA mode (= switching to MSMS mode for eluting peaks). MS2 scans of dominant precursor peaks were acquired and manually analysed with DataAnalysis software version 4.0 (Bruker Daltonik).

### 9. Quantification of mAb content and gp120 binding by ELISA

For determination of PG9 and RSH content, wells were coated with 100 µl of 2 µg/ml anti-human gamma chain (Sigma-Aldrich I3391) in 0.1 N NaHCO₃ buffer (pH 9.6) overnight at 4°C. After washing with PBS containing 0.05% Tween-20 (PBST), samples and ^{CHO}PG9 standards (100 µl) appropriately diluted (1 - 100 ng/ml) in PBST containing 1% BSA were added, the plate was incubated for 60 min, washed and incubated for 60 min with 100 µl of a 1:20.000 dilution of anti-human lambda light chain-peroxidase conjugate (Sigma A5175). After washing with PBST, the wells were incubated for 20 min with 100 µl 3,3',5,5'-tetramethylbenzidine (TMB) substrate solution (Sigma-Aldrich T0440). The reaction was then stopped with 100 µl 30% H₂SO₄ prior to spectrophotometry at 450 nm.

To test binding to gp120, the ELISA setup was adopted as follows: 1 µg/ml gp120^{ZM109} was coated, and the mAb sample concentrations ranged from 10 ng/ml to 4 µg/ml. As detection antibody a 1:2.000 dilution of anti-human IgG (Fcgamma specific)-peroxidase conjugate (Invitrogen 62-8420) was used.

### 10. Biolayer interferometry

PG9 was bound at 20 µg/ml to Dip and Read™ Protein A Biosensor sticks (fortéBio) and antigen binding kinetics were determined with gp120^{ZM109} solutions ranging from 50 µg/ml to 6.25 µg/ml (1:2 dilutions). Blanks were run without PG9 and/or without gp120^{ZM109}. All measurements were conducted at 30°C. Results were analysed with Octet Data Analysis Software 6.4 with single reference-well subtractions. The kinetic constants were computed for each curve separately assuming that dissociation does not reach the pre-association baseline. All estimates with a coefficient of determination (R²) above 0.85 were considered for calculation of the dissociation constant *K*_{d}.

### 11. Virus neutralization assays

Pseudotyped virions were generated as described previously (Gach JS, et al. PLoS One 8(2013):e72054). In brief, 5 × 10⁵ human embryonic kidney 293T cells (ATCC, # CRL-3216) were cotransfected with 4 µg of the HIV Env-deleted backbone plasmid pSG3ΔEnv and 2 µg of the respective Env complementation plasmid using polyethyleneimine (18 µg) as a transfection reagent. Cell culture supernatants were harvested 48 h after transfection, cleared by centrifugation at 4.000 g for 10 min, and then used for single-round infectivity assays as described elsewhere (Gach JS, et al. PLoS One 9(2014):e85371). Briefly, pseudotyped virus was added at a 1:1 volume ratio to serially diluted (1:3) mAbs (starting at 40 µg/ml) and incubated at 37 °C. After 1 h TZM-bl reporter cells (NIH AIDS Reagent Program, # 8129) were added (1:1 by volume) at 1 × 10⁴ cells/well, supplemented with 10 µg/ml DEAE-dextran and then incubated for a further 48 h at 37 °C. Next, the cells were washed, lysed, and developed with luciferase assay reagent according to the manufacturer's instructions (Promega). Relative light units were then measured using a microplate luminometer (BioTek, Synergy 2 luminescence microplate reader). All experiments were performed at least in duplicate. The extent of virus neutralization in the presence of antibody was determined as the 50% or 90% inhibitory concentration (IC₅₀. IC₉₀) as compared to samples treated without mAb.

### 12. Protein quantification

The total protein content of gp120 and mAb samples was quantified with the BCA Protein Assay Kit (Pierce) using BSA as standard according to the protocol provided by the manufacturer.

### Example 1: Natural ^{ΔXF}PG9 binds gp120 less efficiently than ^{CHO}PG9

Previously the *in planta* production of PG9 in ΔXT/FT *Nicotiana benthamiana* plants that have been glycoengineered to remove the plant-typical *N*-glycan residues β1,2-xylose and core α1,3-fucose has been reported (Niemer M, et al. Biotech J 9(2014):493-500). Another recent study has revealed that changing three consecutive amino acids of the PG9 light chain into the corresponding PG16 residues (T^{L94}RR^{L95A} to R^{L94}SH^{L95A}) leads to improved antigen-binding characteristics and higher neutralization efficiency (Pancera M, et al. Nat Struct Mol Biol 20(2013):804-+). Therefore, a ^{ΔXF}PG9-R^{L94}SH^{L95A} variant termed ^{ΔXF}RSH was constructed. Using protein A affinity chromatography, ^{ΔXF}PG9 and ^{ΔXF}RSH could be purified in good yields from leaf extracts. When analyzed by SDS-PAGE under reducing conditions, the ^{ΔXF}PG9 and ^{ΔXF}RSH heavy and light chains showed the expected migration pattern, with the light chains displaying higher electrophoretic mobilities than their CHO-derived counterpart (^{CHO}PG9) due to the removal of a functionally unnecessary *N*-glycosylation site. Under non-reducing conditions, ^{ΔXF}PG9 and ^{ΔXF}RSH yielded single major bands co-migrating with ^{CHO}PG9 (Fig. 2).

To investigate the antigen-binding properties of ^{ΔXF}PG9 in comparison to ^{CHO}PG9, a suitable ligand was required. PG9 has been described to bind with high affinity to trimeric envelope glycoproteins of a wide variety of HIV isolates and also to gp120 monomers of selected HIV strains including ZM109. Therefore gp120^{ZM109} containing a C-terminal hexahistidine tag was expressed in FreeStyle 293 (FS293) cells and purified by metal-chelate affinity chromatography to apparent homogeneity. SDS-PAGE revealed a diffuse band as expected for a heavily glycosylated protein. *N*-glycosylation of two gp120 asparagines (Asn¹⁶⁰ and Asn¹⁷³) has been shown to be important for PG9 binding. Glycosylation analysis by mass spectrometry revealed mainly Man5 structures on either of these *N*-glycosylation sites. Importantly, PG9 is known to prefer such *N*-glycans on Asn¹⁶⁰ while tolerating them on Asn¹⁷³. Only minor amounts of other *N*-glycans were detected on either site, indicating that FS293-derived gp120^{ZM109} meets the prerequisites for a high-affinity PG9 ligand. When tested by ELISA, binding of ^{ΔXF}PG9 to gp120^{ZM109} was found to be considerably weaker than observed for ^{CHO}PG9 (Table A).

**Table A: Sulfation enhances binding of PG9 and RSH to gp120/140. Binding of PG9 and RSH antibodies to immobilized antigen was measured by ELISA in triplicates (monomeric gp120^{ZM109}) or duplicates (trimeric gp140^{BG505.SOSIP.664}). Data are presented as means ± SD.**

| | EC₅₀ [ng/ml] | |
|---|---|---|
| mAb | gp120 | gp140 |
| ^{CHO}PG9 | 89 ± 3 | 290 ± 120 |
| ^{AXF}PG9 | 452 ± 72 | 4870 ± 1560 |
| ^{ΔXF}PG9_{Sulf} | 92 ± 16 | 490 ± 240 |
| ^{ΔXF}PG9_{SulfSia} | 101 ± 12 | 310 ± 50 |
| ^{ΔXF}RSH | 179 ± 85 | 2230 ± 170 |
| ^{ΔXF}RSH_{Sulf} | 82 ± 33 | 180 ± 10 |
| ^{ΔXF}RSH_{SulfSia} | 73 ± 22 | 180 ± 40 |

### Example 2: Co-expression of TPST1 enables the in planta production of sulfated PG9

Sulfation of one or two tyrosine residues in the CDR H3 domain of PG9 and other V1/V2-directed bnAbs has been described to enhance antigen binding. However, a functional tyrosylprotein sulfotransferase is not contained in the *N. benthamiana* genome. Therefore, it is obvious that ^{ΔXF}PG9 was not properly sulfated. Analysis of CDR H3 peptides by mass spectrometry revealed a high degree (82%) of sulfation in the case of ^{CHO}PG9, whereas this post-translational modification was not detected for ^{ΔXF}PG9 (Table B).

**Table B: Tyrosine sulfation of plant-produced PG9 and RSH. The sulfation status of ^{CHO}PG9 is shown for comparison.**

| | **^{CHO}PG9** | **PG9** | **PG9_{Sulf}** | **PG9_{SulfSi a}** | **RSH** | **RSH_{Sulf}** | **RSH_{SulfSia}** |
|---|---|---|---|---|---|---|---|
| **0S [%]** | 18.5 | > 98 | 49.2 | 42.9 | > 98 | 52.7 | 48.9 |
| **1s [%]** | 60.4 | < 1 | 33.2 | 33.8 | < 1 | 30.5 | 34.3 |
| **2S [%]** | 21.1 | < 1 | 17.6 | 23.3 | < 1 | 16.8 | 16.8 |
| **1+2S [%]** | 81.5 | < 2 | 50.8 | 57.1 | < 2 | 47.3 | 51.1 |

Therefore, ^{ΔXF}PG9 was co-expressed with human TPST1 (hsTPST1) in *N. benthamiana.* To mediate proper targeting to sub-Golgi compartments, three constructs carrying different cytoplasmic tail, transmembrane domain and stem (CTS) regions were tested. Expression of hsTPST1 in combination with its authentic CTS region (p^{Fulll}hsTPST1) led to 15-20% sulfated ^{ΔXF}PG9 (Table C). Interestingly, replacement of the natural hsTPST1 CTS region with the corresponding parts of glycosylation enzymes known to be targeted to the trans-region of the plant Golgi (p^{Fut11}hsTPST1 and p^{RST}h-sTPST1) led to a substantially higher level of ^{ΔXF}PG9 sulfation, almost reaching the extent of tyrosine sulfation observed for ^{CHO}PG9. Using p^{RST}hsTPST1, up to 57% of ^{ΔXF}PG9 could be mono- or disulfated (Table B; Table C).

**Table C: Tyrosine sulfation of PG9 and RSH. Relative amounts of unsulfated (0S), singly (1S) and doubly (2S) sulfated PG9 and RSH when coexpressed with different hsTPST1 constructs in plants. The sulfation status of ^{CHO}PG9 is shown for comparison. Data are presented as means ± SD of 2-9 analyses.**

| **mAb** | **TPST1** | **OD₆₀₀** | **0S** | **1S** | **2S** |
|---|---|---|---|---|---|
| | | | | | |
| **^{ΔXF}PG9_{Sulf}** | p^{Full}hsTPST1 | 0.01 | 91.5 ± 7.0 | 8.2 ± 6.4 | 0.3 ± 0.7 |
| **^{ΔXF}PG9_{Sulf}** | p^{Full}hsTPST1 | 0.05 | 83.4 ± 13.9 | 16 ± 13.1 | 0.6 ± 0.9 |
| **^{ΔXF}PG9_{Sulf}** | p^{Full}hsTPST1 | 0.2 | 83.7 ± 8.8 | 15.6 ± 8.2 | 0.7 ± 0.9 |
| **^{ΔXF}PG9_{Sulf}** | p^{Full}hsTPST1 | 0.8 | 82.8 ± 11.7 | 16.8 ± 11 | 0.4 ± 0.7 |
| | | | | | |
| **^{ΔXF}PG9_{Sulf}** | p^{Fut11}hsTPST1 | 0.01 | 73.7 ± 5.8 | 17.8 ± 3.3 | 8.6 ± 2.6 |
| **^{ΔXF}PG9_{Sulf}** | P^{Fut11}hsTPST1 | 0.05 | 57.4 ± 0.8 | 32.1 ± 2.3 | 10.6 ± 3.1 |
| **^{ΔXF}PG9_{Sulf}** | p^{Fut11}hsTPST1 | 0.2 | 59.6 ± 5.8 | 28.1 ± 5.4 | 12.3 ± 5.4 |
| **^{ΔXF}PG9_{Sulf}** | P^{Fut11}hsTPST1 | 0.8 | 58.7 ± 8.7 | 32.7 ± 10.8 | 8.6 ± 2.1 |
| | | | | | |
| **^{ΔXF}PG9_{Sulf}** | p^{RST}hsTPST1 | 0.01 | 69.5 ± 8.9 | 20.6 ± 6.9 | 9.9 ± 2.3 |
| **^{ΔXF}PG9_{Sulf}** | p^{RST}hsTPST1 | 0.05 | 53.9 ± 6.1 | 30.8 ± 2.3 | 15.3 ± 3.8 |
| **^{ΔXF}PG9_{Sulf}** | p^{RST}hsTPST1 | 0.2 | 57.2 ± 10.4 | 29.3 ± 7.4 | 13.5 ± 5.1 |
| **^{ΔXF}PG9_{Sulf}** | p^{RST}hsTPST1 | 0.8 | 53.7 ± 11.1 | 36.8 ± 11.8 | 9.5 ± 2.7 |
| **^{ΔXF}RSH_{Sulf}** | p^{RST}hsTPST1 | 0.2 | 57.4 ± 7.6 | 29.6 ± 5.2 | 13.1 ± 3.6 |
| | | | | | |
| **^{CHO}PG9** | | | 18.5 ± 1.5 | 60.4 ± 3.9 | 21.1 ± 5.4 |

### Example 3: Tyrosine sulfation of PG9 produced in plants and CHO cells occurs at the same positions

The tryptic CDR H3 peptide used for analyzing the PG9 sulfation status by LC-ESI-MS (N^{100C}GYNYYDFYDGYYNYHYMDVWGK¹⁰⁵; SEQ ID No. 27) contains several tyrosine residues that are potential TPST targets. To narrow down which amino acids are sulfated in the case of plant-derived PG9, the tryptic peptide was further digested with AspN to give rise to the shorter peptide N^{100C}GYNYY^{100H} (SEQ ID No. 28), containing the tyrosines involved in gp120 binding, as well as D^{100I}FYDGYYNYHYM^{100T} (SEQ ID No. 29) and D¹⁰¹WGK¹⁰⁵. In plant-produced as well as CHO-derived PG9 and RSH, no sulfates were found on D^{100I}FYDGYYNYHYM^{100T} (SEQ ID No. XX) (Fig. 3), whereas N^{100C}GYNYY^{100H} (SEQ ID No. 28) was found to be singly and doubly sulfated to roughly the same extent as N^{100C}GYNYYDFYDGYYNYHYMDVWGK¹⁰⁵ (SEQ ID No. 27). This indicates that the sulfate groups are attached to Y^{100E}, Y^{100G} and/or Y^{100H} independent of the expression platform used for PG9 production. It has been shown previously by X-ray crystallography that Y^{100G} and Y^{100H} of mammalian cell-produced PG9 can be sulfated. This shows that human TPST1 also modifies the same tyrosine residues *in planta.*

### Example 4: PG9 carries human-type N-glycans when expressed in glycoengineered plants

Mass spectrometric *N*-glycan analysis of ^{ΔXF}PG9, ^{ΔXF}PG9_{Sulf}, ^{ΔXF}RSH and ^{ΔXF}RSH_{Sulf} revealed the presence of a single dominant N-glycan species, GnGn (GO). This glycoform accounted for roughly 45-50% of all *N*-glycan species. Upon coexpression of PG9 and RSH with mammalian genes necessary for terminal galactosylation and sialylation *in planta* resulting in the synthesis of ^{ΔXF}PG9_{SulfSia} and ^{ΔXF}RSH_{SulfSia}, the N-glycosylation profiles shifted to 30-40% galactosylated oligosaccharides and 6-12% sialylated glycans, with G0 reduced to 15-20%. Importantly, core α1,3-fucose and β1,2-xylose residues were barely detectable in all 6 plant-produced variants (below 5%). In the case of ^{CHO}PG9, the vast majority of Asn²⁹⁷ *N*-glycans contained α1,6-fucose (more than 95%) and the main *N*-glycan structure detected (70%) was G0F⁶ (GnGnF⁶). Roughly 20% of ^{CHO}PG9 was galactosylated and less than 1% sialylated. These results indicate that the *N*-glycan moieties of ^{ΔXF}PG9_{SulfSia} and ^{ΔXF}RSH_{SulfSia} are largely devoid of the core fucose residues known to hamper mAb binding to Fc receptors while otherwise being reminiscent of those found on PG9 produced in mammalian cell factories.

### Example 5: Antigen binding by ^{ΔXF}PG9 is enhanced by tyrosine sulfation

Binding of the different PG9 and RSH variants to monomeric gp120^{ZM109} or trimeric gp140^{BG505.SOSIP.664} was tested by ELISA (Table A), and EC₅₀ values were calculated. RSH showed up to 3-fold better binding to either antigen than PG9. Sulfation of plant-produced PG9 and RSH increased their affinities 10-16 times for trimeric gp140 and 2-5 times for monomeric gp120^{ZM109}. As expected, different glycoforms showed very similar EC₅₀ values. The avidity of the antigen-antibody interaction was also determined by biolayer interferometry (Table D).

**Table D: Affinities of PG9 and RSH for gp120^{ZM109} as determined by biolayer interferometry measurements. Data are presented as means ± SD of 2 (^{ΔXF}RSH) or 4-6 individual determinations. The binding of ^{ΔXF}PG9 to gp120^{ZM109} was too weak for accurate determination of K_{d} under the experimental conditions used.**

| | *K*_{d} [nM] |
|---|---|
| ^{CHO}PG9 | 756 ± 365 |
| ^{ΔXF}PG9_{Sulf} | 525 ± 167 |
| ^{ΔXF}RSH_{Sulf} | 605 ± 239 |
| ^{ΔXF}RSH | 2510 ± 39 |
| | |
| ^{ΔXF}PG9 | >3000 |

^{ΔXF}PG9_{Sulf}, ^{ΔXF}RSH_{Sulf} and ^{CHO}PG9 showed roughly the same affinity to the antigen (*K*_{d} of 525, 605 and 756 nM, respectively), whereas unsulfated ^{ΔXF}RSH exhibited a roughly 4-fold lower affinity (*K*_{d} of 2.51 µM). The results obtained by biolayer interferometry confirmed those from the ELISA experiments, namely that RSH binds stronger to gp120/gp140 than wild-type PG9 and that tyrosine sulfation increases the affinity of both antibodies for either antigen. Furthermore, ^{ΔXF}PG9_{Sulf} and ^{ΔXF}PG9_{SulfSial} displayed essentially the same gp120/140-binding properties as ^{CHO}PG9, demonstrating the suitability of our plant-based expression platform to produce fully active versions of this bnAb.

### Example 6: Increased virus neutralization by sulfated PG9 and RSH variants

Finally, the neutralization efficiencies of the antibodies on a panel of HIV clade B and clade C pseudoviruses were tested (Table E).

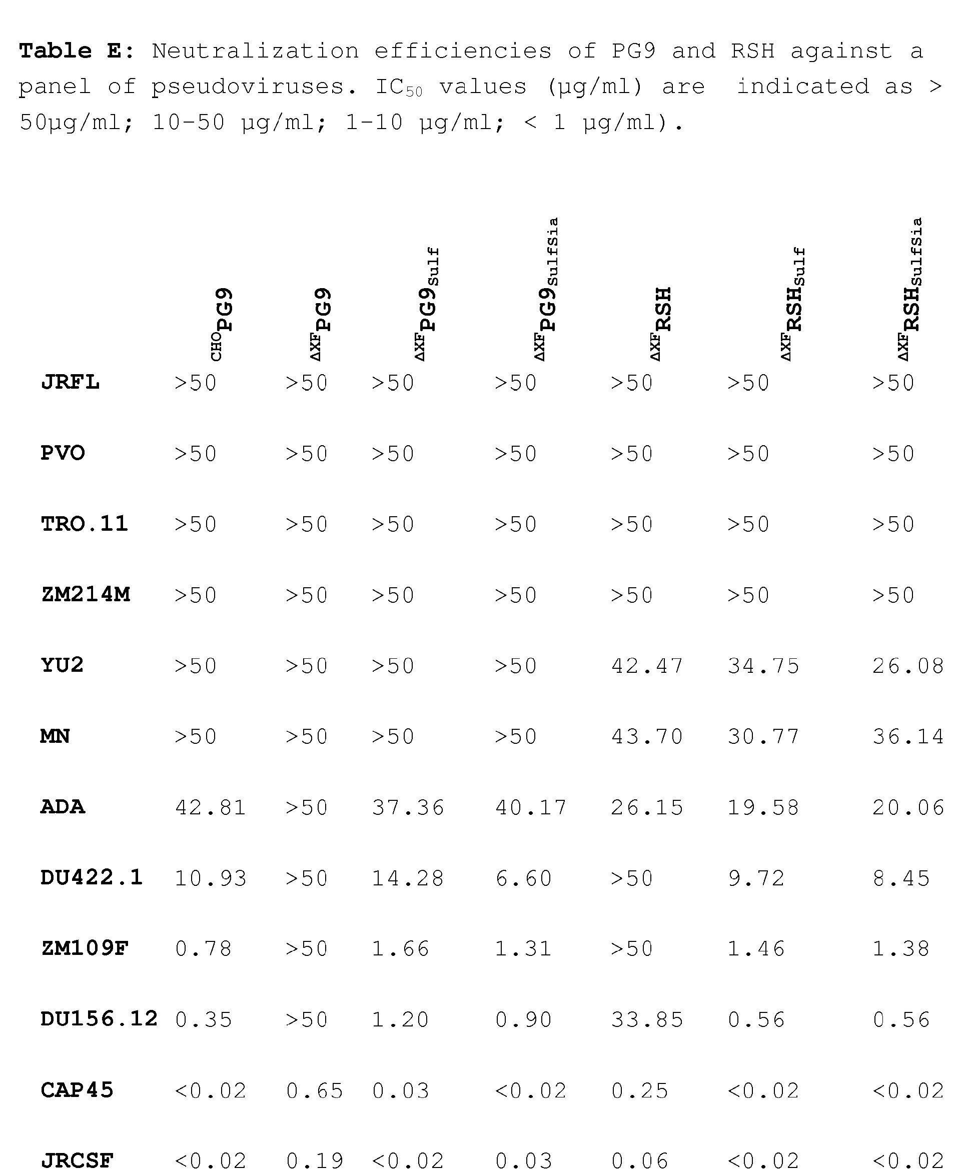

The viruses included well-neutralized isolates as well as some resistant to PG9 or RSH produced in mammalian cells. As expected, a number of pseudoviruses was not neutralized under the tested conditions (JRFL, ZM214M, PVO, TRO.11), whereas others were neutralized at intermediate (ADA, YU2, MN) to good efficiency (DU156.12, DU422.1, ZM109) and some were neutralized very efficiently (JRCSF, CAP45). Interestingly, the various PG9 variants displayed pronounced differences with respect to their neutralization efficiencies. In accordance with the results of the antigen-binding assays, tyrosine sulfation strongly enhanced neutralization of highly sensitive isolates (50-fold and more; e.g. JRCSF, DU156.12, ZM109, CAP45, DU422.1), whereas only a modest improvement was observed for more resistant strains (1.3-1.5 fold; ADA, YU2 and MN). These data provide unprecedented evidence for the pivotal role of CDR H3 sulfotyrosines in effective HIV neutralization by PG9 as previously proposed based on the tertiary structure of the PG9/gp120 complex. In general, the varying sensitivities of the tested HIV strains to PG9 and RSH were in good agreement with the presence or absence of PG9-interacting residues in their gp120 V2 sequences. Furthermore, the observed differences in neutralization efficiency between PG9 and RSH were comparable to those found in antigen-binding assays. Importantly, glycoengineering of plant-derived PG9 and RSH did not affect virus neutralization, thus demonstrating that fine tuning of Asn²⁹⁷ *N*-glycosylation does not compromise the anti-viral potency of these bnAbs.

### Conclusion:

The examples provided herein aimed at the establishment of sulfoengineering in plants in order to increase the *in vivo* efficiency of two HIV-specific mAbs, PG9 and RSH. Although some plants have a tyrosylprotein sulfotransferase and can sulfate phytohormones, PG9 expressed in *N. benthamiana* did not contain detectable amounts of sulfated peptides, indicating that mammalian-type sulfation does not occur naturally in *N. benthamiana* leaves. In humans, sulfation of suitable tyrosine residues is carried out by the two tyrosylprotein sulfotransferases hsTPST1 and hsTPST2, and overexpression of hsTPST1 and hsTPST2 has been shown to increase sulfation of recombinantly produced proteins in CHO and HEK293T cells. However, expression of full-length hsTPST1 did not yield high levels of sulfation in *N. benthamiana.* Replacing the authentic CTS sequence of hsTPST1 with a plant CTS region, for instance, drastically increased the sulfation efficiency and led to mAbs with an increased neutralization efficacy.

The crystal structure of PG9 in complex with its antigen has revealed that Y^{100H} and Y^{100G} of the PG9 (and RSH) heavy chain can be sulfated and that their sulfation increases antigen binding. By mass spectrometry, the sulfation sites of ^{CHO}PG9 and plant-produced PG9/RSH could be mapped to a short peptide containing 3 tyrosine residues (Y^{100E}, Y^{100H} and Y^{100G}). Sulfation of plant-produced PG9 and RSH enhanced antigen binding and virus neutralization, indicating that also in plants Y^{100H} and Y^{100G} are the sulfated residues. While the impact of tyrosine sulfation on neutralization efficiency was so far only assessed for singly and doubly sulfated PG9, sulfated and unmodified PG9/RSH were compared and a far more pronounced difference in anti-viral potency was observed. These results show that singly sulfated PG9 binds and neutralizes HIV better than non-sulfated antibody, and that the doubly sulfated mAb has an even higher efficacy.

## Claims

1. A nucleic acid molecule encoding
a) a modified tyrosylprotein sulfotransferase of a wild-type tyrosylprotein sulfotransferase, wherein the cytoplasmic transmembrane stem (CTS) region of the wild-type tyrosylprotein sulfotransferase is replaced by a heterologous CTS region, or
b) a fusion protein comprising a catalytically active fragment of a tyrosylprotein sulfotransferase fused to a heterologous CTS region.

2. Nucleic acid molecule according to claim 1, wherein the heterologous CTS region is a plant or animal CTS region.

3. Nucleic acid molecule according to claim 1 or 2, wherein the heterologous CTS region is a CTS region of a glycosyltransferase or a tyrosylprotein sulfotransferase.

4. Nucleic acid molecule according to claim 3, wherein the glycosyltransferase is selected from the group consisting of a fucosyltransferase, preferably an α1,3-fucosyltransferase, more preferably an α1,3-Fucosyltransferase 11, and a sialytransferase, preferably an α2,6-sialytransferase.

5. Nucleic acid molecule according to any one of claims 1 to 4, wherein the tyrosylprotein sulfotransferase is a plant or animal tyrosylprotein sulfotransferase.

6. Nucleic acid molecule according to any one of claims 1 to 5, wherein the heterologous CTS region of said fusion protein is Nor C-terminally, preferably N-terminally, fused to the tyrosylprotein sulfotransferase or the catalytically active fragment thereof.

7. Polypeptide encoded by a nucleic acid molecule according to any one of claims 1 to 6.

8. A vector comprising a nucleic acid molecule according to any one of claims 1 to 6.

9. A plant or plant cell capable of transferring a sulfate moiety to a tyrosine residue of a polypeptide heterologously produced in said plant or plant cell comprising a nucleic acid molecule according to any one of claims 1 to 6 or a vector according to claim 8.

10. Plant or plant cell according to claim 9, wherein said transgenic plant or plant cell comprises further a nucleic acid molecule encoding a heterologous polypeptide operably linked to a promoter region.

11. Plant or plant cell according to claim 10, wherein the heterologous polypeptide is of animal origin, preferably a mammalian, more preferably a human, polypeptide.

12. Plant or plant cell according to claim 11, wherein the heterologous animal polypeptide is an antibody.

13. Plant or plant cell according to claim 12, wherein the antibody is an antibody binding to an HIV surface protein.

14. Plant or plant cell according to claim 12 or 13, wherein the antibody is an antibody selected from the group consisting of PG9, PG16, PGT141-145, 47e, 412d, Sb1, C12, E51, CM51 and a variant thereof.

15. Plant or plant cell according to claim 14, wherein the antibody variant is a PG9 antibody comprising modifications at R^{L94}SH^{L95A}.

16. A method of recombinantly producing a polypeptide of animal origin carrying an animal-type sulfation comprising the step of cultivating a plant or plant cell according to any one of claims 9 to 15.


SEQUENCE LISTING

<110>  Universität für Bodenkultur

<120>  Nucleic acid molecule and uses thereof

<130>  47139

<160>  35

<170>  PatentIn version 3.5

<210>  1
<211>  370
<212>  PRT
<213>  Homo sapiens

<400>  1

Met Val Gly Lys Leu Lys Gln Asn Leu Leu Leu Ala Cys Leu Val Ile
1               5                   10                  15

Ser Ser Val Thr Val Phe Tyr Leu Gly Gln His Ala Met Glu Cys His
            20                  25                  30

His Arg Ile Glu Glu Arg Ser Gln Pro Val Lys Leu Glu Ser Thr Arg
        35                  40                  45

Thr Thr Val Arg Thr Gly Leu Asp Leu Lys Ala Asn Lys Thr Phe Ala
    50                  55                  60

Tyr His Lys Asp Met Pro Leu Ile Phe Ile Gly Gly Val Pro Arg Ser
65                  70                  75                  80

Gly Thr Thr Leu Met Arg Ala Met Leu Asp Ala His Pro Asp Ile Arg
                85                  90                  95

Cys Gly Glu Glu Thr Arg Val Ile Pro Arg Ile Leu Ala Leu Lys Gln
            100                 105                 110

Met Trp Ser Arg Ser Ser Lys Glu Lys Ile Arg Leu Asp Glu Ala Gly
        115                 120                 125

Val Thr Asp Glu Val Leu Asp Ser Ala Met Gln Ala Phe Leu Leu Glu
    130                 135                 140

Ile Ile Val Lys His Gly Glu Pro Ala Pro Tyr Leu Cys Asn Lys Asp
145                 150                 155                 160

Pro Phe Ala Leu Lys Ser Leu Thr Tyr Leu Ser Arg Leu Phe Pro Asn
                165                 170                 175

Ala Lys Phe Leu Leu Met Val Arg Asp Gly Arg Ala Ser Val His Ser
            180                 185                 190

Met Ile Ser Arg Lys Val Thr Ile Ala Gly Phe Asp Leu Asn Ser Tyr
        195                 200                 205

Arg Asp Cys Leu Thr Lys Trp Asn Arg Ala Ile Glu Thr Met Tyr Asn
    210                 215                 220

Gln Cys Met Glu Val Gly Tyr Lys Lys Cys Met Leu Val His Tyr Glu
225                 230                 235                 240

Gln Leu Val Leu His Pro Glu Arg Trp Met Arg Thr Leu Leu Lys Phe
                245                 250                 255

Leu Gln Ile Pro Trp Asn His Ser Val Leu His His Glu Glu Met Ile
            260                 265                 270

Gly Lys Ala Gly Gly Val Ser Leu Ser Lys Val Glu Arg Ser Thr Asp
        275                 280                 285

Gln Val Ile Lys Pro Val Asn Val Gly Ala Leu Ser Lys Trp Val Gly
    290                 295                 300

Lys Ile Pro Pro Asp Val Leu Gln Asp Met Ala Val Ile Ala Pro Met
305                 310                 315                 320

Leu Ala Lys Leu Gly Tyr Asp Pro Tyr Ala Asn Pro Pro Asn Tyr Gly
                325                 330                 335

Lys Pro Asp Pro Lys Ile Ile Glu Asn Thr Arg Arg Val Tyr Lys Gly
            340                 345                 350

Glu Phe Gln Leu Pro Asp Phe Leu Lys Glu Lys Pro Gln Thr Glu Gln
        355                 360                 365

Val Glu
    370

<210>  2
<211>  1113
<212>  DNA
<213>  Homo sapiens

<400>  2
atggttggaa agctgaagca gaacttacta ttggcatgtc tggtgattag ttctgtgact        60

gtgttttacc tgggccagca tgccatggaa tgccatcacc ggatagagga acgtagccag       120

ccagtcaaat tggagagcac aaggaccact gtgagaactg gcctggacct caaagccaac       180

aaaacctttg cctatcacaa agatatgcct ttaatattta ttggaggtgt gcctcggagt 240

ggaaccacac tcatgagggc catgctggac gcacatcctg acattcgctg tggagaggaa 300

accagggtca ttccccgaat cctggccctg aagcagatgt ggtcacggtc aagtaaagag 360

aagatccgcc tggatgaggc tggtgttact gatgaagtgc tggattctgc catgcaagcc 420

ttcttactag aaattatcgt taagcatggg gagccagccc cttatttatg taataaagat 480

ccttttgccc tgaaatcttt aacttacctt tctaggttat tccccaatgc caaatttctc 540

ctgatggtcc gagatggccg ggcatcagta cattcaatga tttctcgaaa agttactata 600

gctggatttg atctgaacag ctatagggac tgtttgacaa agtggaatcg tgctatagag 660

accatgtata accagtgtat ggaggttggt tataaaaagt gcatgttggt tcactatgaa 720

caacttgtct tacatcctga acggtggatg agaacactct taaagttcct ccagattcca 780

tggaaccact cagtattgca ccatgaagag atgattggga aagctggggg agtgtctctg 840

tcaaaagtgg agagatctac agaccaagta atcaagccag tcaatgtagg agctctatca 900

aaatgggttg ggaagatacc gccagatgtt ttacaagaca tggcagtgat tgctcctatg 960

cttgccaagc ttggatatga cccatatgcc aacccaccta actacggaaa acctgatccc 1020

aaaattattg aaaacactcg aagggtctat aagggagaat tccaactacc tgactttctt 1080

aaagaaaaac cacagactga gcaagtggag tag 1113

<210> 3
<211> 39
<212> PRT
<213> Artificial Sequence

<220>
<223> CTS region of SEQ ID No. 1

<400> 3

Met Val Gly Lys Leu Lys Gln Asn Leu Leu Leu Ala Cys Leu Val Ile
1               5                   10                  15

Ser Ser Val Thr Val Phe Tyr Leu Gly Gln His Ala Met Glu Cys His
            20                  25                  30

His Arg Ile Glu Glu Arg Ser
        35

<210> 4
<211> 117
<212> DNA
<213> Artificial Sequence

<220>
<223> Nucleic acid molecule encoding a CTS region

<400>  4
atggttggaa agctgaagca gaacttacta ttggcatgtc tggtgattag ttctgtgact      60

gtgttttacc tgggccagca tgccatggaa tgccatcacc ggatagagga acgtagc        117


<210>  5
<211>  66
<212>  PRT
<213>  Artificial Sequence

<220>
<223>  CTS region

<400>  5

Met Gly Val Phe Ser Asn Leu Arg Gly Pro Lys Ile Gly Leu Thr His
1               5                   10                  15


Glu Glu Leu Pro Val Val Ala Asn Gly Ser Thr Ser Ser Ser Ser Ser
            20                  25                  30


Pro Ser Ser Phe Lys Arg Lys Val Ser Thr Phe Leu Pro Ile Cys Val
        35                  40                  45


Ala Leu Val Val Ile Ile Glu Ile Gly Phe Leu Cys Arg Leu Asp Asn
    50                  55                  60


Ala Ser
65


<210>  6
<211>  198
<212>  DNA
<213>  Artificial Sequence

<220>
<223>  Nucleic acid sequence encoding a CTS region

<400>  6
atgggtgttt tctccaatct tcgaggtcct aaaattggat tgacccatga agaattgcct      60

gtagtagcca atggctctac ttcttcttct tcgtctcctt cctctttcaa gcgtaaagtc     120

tcgacctttt tgccaatctg cgtggctctt gtcgtcatta tcgagatcgg gttcctctgt     180

cggctcgata acgcttct                                                   198


<210>  7
<211>  52
<212>  PRT
<213>  Artificial Sequence

<220>
<223>  CTS region

<400>  7

Met Ile His Thr Asn Leu Lys Lys Lys Phe Ser Leu Phe Ile Leu Val
1               5                   10                  15

Phe Leu Leu Phe Ala Val Ile Cys Val Trp Lys Lys Gly Ser Asp Tyr
            20                  25                  30

Glu Ala Leu Thr Leu Gln Ala Lys Glu Phe Gln Met Pro Lys Ser Gln
        35                  40                  45

Glu Lys Val Ala
    50

<210>  8
<211>  156
<212>  DNA
<213>  Artificial Sequence

<220>
<223>  Nucleic acid sequence encoding a CTS region

<400>  8
atgattcata ccaacttgaa gaaaaagttc agcctcttca tcctggtctt tctcctgttc      60

gcagtcatct gtgtttggaa gaaagggagc gactatgagg cccttacact gcaagccaag     120

gaattccaga tgcccaagag ccaggagaaa gtggcc                               156

<210>  9
<211>  1195
<212>  DNA
<213>  Artificial Sequence

<220>
<223>  Nucleic acid encoding a human tyrosylprotein sulfotransferase
       whose wild-type CTS region at its N-terminus has been replaced by
       a CTS region of alpha-1,3-Fucosyltransferase 11 of Arabidopsis
       thaliana

<220>
<221>  misc_feature
<222>  (199)..(199)
<223>  n=1 to 10 nucleic acid resiudes

<400>  9
atgggtgttt tctccaatct tcgaggtcct aaaattggat tgacccatga agaattgcct      60

gtagtagcca atggctctac ttcttcttct tcgtctcctt cctctttcaa gcgtaaagtc     120

tcgacctttt tgccaatctg cgtggctctt gtcgtcatta tcgagatcgg gttcctctgt     180

cggctcgata acgcttctnc agccagtcaa attggagagc acaaggacca ctgtgagaac     240

tggcctggac ctcaaagcca acaaaacctt tgcctatcac aaagatatgc ctttaatatt     300

tattggaggt gtgcctcgga gtggaaccac actcatgagg gccatgctgg acgcacatcc     360

tgacattcgc tgtggagagg aaaccagggt cattccccga atcctggccc tgaagcagat 420

gtggtcacgg tcaagtaaag agaagatccg cctggatgag gctggtgtta ctgatgaagt 480

gctggattct gccatgcaag ccttcttact agaaattatc gttaagcatg gggagccagc 540

cccttattta tgtaataaag atccttttgc cctgaaatct ttaacttacc tttctaggtt 600

attccccaat gccaaatttc tcctgatggt ccgagatggc cgggcatcag tacattcaat 660

gatttctcga aaagttacta tagctggatt tgatctgaac agctataggg actgtttgac 720

aaagtggaat cgtgctatag agaccatgta taaccagtgt atggaggttg gttataaaaa 780

gtgcatgttg gttcactatg aacaacttgt cttacatcct gaacggtgga tgagaacact 840

cttaaagttc ctccagattc catggaacca ctcagtattg caccatgaag agatgattgg 900

gaaagctggg ggagtgtctc tgtcaaaagt ggagagatct acagaccaag taatcaagcc 960

agtcaatgta ggagctctat caaaatgggt tgggaagata ccgccagatg ttttacaaga 1020

catggcagtg attgctccta tgcttgccaa gcttggatat gacccatatg ccaacccacc 1080

taactacgga aaacctgatc ccaaaattat tgaaaacact cgaagggtct ataagggaga 1140

attccaacta cctgactttc ttaaagaaaa accacagact gagcaagtgg agtag 1195

<210> 10
<211> 1153
<212> DNA
<213> Artificial Sequence

<220>
<223> Nucleic acid sequence encoding a human tyrosylprotein
 sulfotransferase whose wild-type CTS region at its N-terminus has
 been replaced by a CTS region of an alpha-2,6-sialytransferase of
 Rattus norvegicus

<220>
<221> misc_feature
<222> (157)..(157)
<223> n=1 to 10 nucleic acid resiudes

<400> 10
atgattcata ccaacttgaa gaaaaagttc agcctcttca tcctggtctt tctcctgttc 60

gcagtcatct gtgtttggaa gaaagggagc gactatgagg cccttacact gcaagccaag 120

gaattccaga tgcccaagag ccaggagaaa gtggccncag ccagtcaaat tggagagcac 180

aaggaccact gtgagaactg gcctggacct caaagccaac aaaacctttg cctatcacaa 240

agatatgcct ttaatattta ttggaggtgt gcctcggagt ggaaccacac tcatgagggc 300

catgctggac gcacatcctg acattcgctg tggagaggaa accagggtca ttccccgaat 360

cctggccctg aagcagatgt ggtcacggtc aagtaaagag aagatccgcc tggatgaggc 420

tggtgttact gatgaagtgc tggattctgc catgcaagcc ttcttactag aaattatcgt 480

taagcatggg gagccagccc cttatttatg taataaagat ccttttgccc tgaaatcttt 540

aacttacctt tctaggttat tccccaatgc caaatttctc ctgatggtcc gagatggccg 600

ggcatcagta cattcaatga tttctcgaaa agttactata gctggatttg atctgaacag 660

ctatagggac tgtttgacaa agtggaatcg tgctatagag accatgtata accagtgtat 720

ggaggttggt tataaaaagt gcatgttggt tcactatgaa caacttgtct tacatcctga 780

acggtggatg agaacactct taaagttcct ccagattcca tggaaccact cagtattgca 840

ccatgaagag atgattggga aagctggggg agtgtctctg tcaaaagtgg agagatctac 900

agaccaagta atcaagccag tcaatgtagg agctctatca aaatgggttg ggaagatacc 960

gccagatgtt ttacaagaca tggcagtgat tgctcctatg cttgccaagc ttggatatga 1020

cccatatgcc aacccaccta actacggaaa acctgatccc aaaattattg aaaacactcg 1080

aagggtctat aagggagaat tccaactacc tgactttctt aaagaaaaac cacagactga 1140

gcaagtggag tag 1153

<210> 11
<211> 398
<212> PRT
<213> Artificial Sequence

<220>
<223> Human tyrosylprotein sulfotransferase whose wild-type CTS region
 at its N-terminus has been replaced by a CTS region of an
 alpha-1,3-Fucosyltransferase 11

<220>
<221> MISC_FEATURE
<222> (67)..(67)
<223> Xaa=1 to 5 amino acid residues

<400> 11

Met Gly Val Phe Ser Asn Leu Arg Gly Pro Lys Ile Gly Leu Thr His
1               5                   10                  15

Glu Glu Leu Pro Val Val Ala Asn Gly Ser Thr Ser Ser Ser Ser Ser
            20                  25                  30

Pro Ser Ser Phe Lys Arg Lys Val Ser Thr Phe Leu Pro Ile Cys Val
        35                  40                  45

Ala Leu Val Val Ile Ile Glu Ile Gly Phe Leu Cys Arg Leu Asp Asn
    50                  55                  60

Ala Ser Xaa Gln Pro Val Lys Leu Glu Ser Thr Arg Thr Thr Val Arg
65                  70                  75                  80

Thr Gly Leu Asp Leu Lys Ala Asn Lys Thr Phe Ala Tyr His Lys Asp
                85                  90                  95

Met Pro Leu Ile Phe Ile Gly Gly Val Pro Arg Ser Gly Thr Thr Leu
            100                 105                 110

Met Arg Ala Met Leu Asp Ala His Pro Asp Ile Arg Cys Gly Glu Glu
        115                 120                 125

Thr Arg Val Ile Pro Arg Ile Leu Ala Leu Lys Gln Met Trp Ser Arg
    130                 135                 140

Ser Ser Lys Glu Lys Ile Arg Leu Asp Glu Ala Gly Val Thr Asp Glu
145                 150                 155                 160

Val Leu Asp Ser Ala Met Gln Ala Phe Leu Leu Glu Ile Ile Val Lys
                165                 170                 175

His Gly Glu Pro Ala Pro Tyr Leu Cys Asn Lys Asp Pro Phe Ala Leu
            180                 185                 190

Lys Ser Leu Thr Tyr Leu Ser Arg Leu Phe Pro Asn Ala Lys Phe Leu
        195                 200                 205

Leu Met Val Arg Asp Gly Arg Ala Ser Val His Ser Met Ile Ser Arg
    210                 215                 220

Lys Val Thr Ile Ala Gly Phe Asp Leu Asn Ser Tyr Arg Asp Cys Leu
225                 230                 235                 240

Thr Lys Trp Asn Arg Ala Ile Glu Thr Met Tyr Asn Gln Cys Met Glu
                245                 250                 255

Val Gly Tyr Lys Lys Cys Met Leu Val His Tyr Glu Gln Leu Val Leu
            260                 265                 270

His Pro Glu Arg Trp Met Arg Thr Leu Leu Lys Phe Leu Gln Ile Pro
        275                 280                 285

Trp Asn His Ser Val Leu His His Glu Glu Met Ile Gly Lys Ala Gly
    290                 295                 300

Gly Val Ser Leu Ser Lys Val Glu Arg Ser Thr Asp Gln Val Ile Lys
305                 310                 315                 320

Pro Val Asn Val Gly Ala Leu Ser Lys Trp Val Gly Lys Ile Pro Pro
                325                 330                 335

Asp Val Leu Gln Asp Met Ala Val Ile Ala Pro Met Leu Ala Lys Leu
            340                 345                 350

Gly Tyr Asp Pro Tyr Ala Asn Pro Pro Asn Tyr Gly Lys Pro Asp Pro
        355                 360                 365

Lys Ile Ile Glu Asn Thr Arg Arg Val Tyr Lys Gly Glu Phe Gln Leu
    370                 375                 380

Pro Asp Phe Leu Lys Glu Lys Pro Gln Thr Glu Gln Val Glu
385                 390                 395

<210>  12
<211>  384
<212>  PRT
<213>  Artificial Sequence

<220>
<223>  Human tyrosylprotein sulfotransferase whose wild-type CTS region
       at its N-terminus has been replaced by a CTS region of an
       alpha-2,6-sialytransferase of Rattus norvegicus

<220>
<221>  MISC_FEATURE
<222>  (53)..(53)
<223>  Xaa=1 to 5 amino acid residues

<400>  12

Met Ile His Thr Asn Leu Lys Lys Lys Phe Ser Leu Phe Ile Leu Val
1               5                   10                  15

Phe Leu Leu Phe Ala Val Ile Cys Val Trp Lys Lys Gly Ser Asp Tyr
            20                  25                  30

Glu Ala Leu Thr Leu Gln Ala Lys Glu Phe Gln Met Pro Lys Ser Gln
        35                  40                  45

Glu Lys Val Ala Xaa Gln Pro Val Lys Leu Glu Ser Thr Arg Thr Thr
    50                  55                  60

Val Arg Thr Gly Leu Asp Leu Lys Ala Asn Lys Thr Phe Ala Tyr His
65                  70                  75                  80

Lys Asp Met Pro Leu Ile Phe Ile Gly Gly Val Pro Arg Ser Gly Thr
                85                  90                  95

Thr Leu Met Arg Ala Met Leu Asp Ala His Pro Asp Ile Arg Cys Gly
            100                 105                 110

Glu Glu Thr Arg Val Ile Pro Arg Ile Leu Ala Leu Lys Gln Met Trp
        115                 120                 125

Ser Arg Ser Ser Lys Glu Lys Ile Arg Leu Asp Glu Ala Gly Val Thr
    130                 135                 140

Asp Glu Val Leu Asp Ser Ala Met Gln Ala Phe Leu Leu Glu Ile Ile
145                 150                 155                 160

Val Lys His Gly Glu Pro Ala Pro Tyr Leu Cys Asn Lys Asp Pro Phe
                165                 170                 175

Ala Leu Lys Ser Leu Thr Tyr Leu Ser Arg Leu Phe Pro Asn Ala Lys
            180                 185                 190

Phe Leu Leu Met Val Arg Asp Gly Arg Ala Ser Val His Ser Met Ile
        195                 200                 205

Ser Arg Lys Val Thr Ile Ala Gly Phe Asp Leu Asn Ser Tyr Arg Asp
    210                 215                 220

Cys Leu Thr Lys Trp Asn Arg Ala Ile Glu Thr Met Tyr Asn Gln Cys
225                 230                 235                 240

Met Glu Val Gly Tyr Lys Lys Cys Met Leu Val His Tyr Glu Gln Leu
                245                 250                 255

Val Leu His Pro Glu Arg Trp Met Arg Thr Leu Leu Lys Phe Leu Gln
            260                 265                 270

Ile Pro Trp Asn His Ser Val Leu His His Glu Glu Met Ile Gly Lys
        275                 280                 285

Ala Gly Gly Val Ser Leu Ser Lys Val Glu Arg Ser Thr Asp Gln Val
    290                 295                 300

Ile Lys Pro Val Asn Val Gly Ala Leu Ser Lys Trp Val Gly Lys Ile
305                 310                 315                 320

Pro Pro Asp Val Leu Gln Asp Met Ala Val Ile Ala Pro Met Leu Ala
                325                 330                 335

Lys Leu Gly Tyr Asp Pro Tyr Ala Asn Pro Pro Asn Tyr Gly Lys Pro
            340                 345                 350

Asp Pro Lys Ile Ile Glu Asn Thr Arg Arg Val Tyr Lys Gly Glu Phe
        355                 360                 365

Gln Leu Pro Asp Phe Leu Lys Glu Lys Pro Gln Thr Glu Gln Val Glu
    370                 375                 380

<210> 13
<211> 377
<212> PRT
<213> Homo sapiens

<400> 13

Met Arg Leu Ser Val Arg Arg Val Leu Leu Ala Ala Gly Cys Ala Leu
1               5                   10                  15

Val Leu Val Leu Ala Val Gln Leu Gly Gln Gln Val Leu Glu Cys Arg
            20                  25                  30

Ala Val Leu Ala Gly Leu Arg Ser Pro Arg Gly Ala Met Arg Pro Glu
        35                  40                  45

Gln Glu Glu Leu Val Met Val Gly Thr Asn His Val Glu Tyr Arg Tyr
    50                  55                  60

Gly Lys Ala Met Pro Leu Ile Phe Val Gly Gly Val Pro Arg Ser Gly
65                  70                  75                  80

Thr Thr Leu Met Arg Ala Met Leu Asp Ala His Pro Glu Val Arg Cys
                85                  90                  95

Gly Glu Glu Thr Arg Ile Ile Pro Arg Val Leu Ala Met Arg Gln Ala
            100                 105                 110

Trp Ser Lys Ser Gly Arg Glu Lys Leu Arg Leu Asp Glu Ala Gly Val
        115                 120                 125

Thr Asp Glu Val Leu Asp Ala Ala Met Gln Ala Phe Ile Leu Glu Val
    130                 135                 140

Ile Ala Lys His Gly Glu Pro Ala Arg Val Leu Cys Asn Lys Asp Pro
145                 150                 155                 160

Phe Thr Leu Lys Ser Ser Val Tyr Leu Ser Arg Leu Phe Pro Asn Ser
                165                 170                 175

Lys Phe Leu Leu Met Val Arg Asp Gly Arg Ala Ser Val His Ser Met
            180                 185                 190

Ile Thr Arg Lys Val Thr Ile Ala Gly Phe Asp Leu Ser Ser Tyr Arg
        195                 200                 205

Asp Cys Leu Thr Lys Trp Asn Lys Ala Ile Glu Val Met Tyr Ala Gln
    210                 215                 220

Cys Met Glu Val Gly Lys Glu Lys Cys Leu Pro Val Tyr Tyr Glu Gln
225                 230                 235                 240

Leu Val Leu His Pro Arg Arg Ser Leu Lys Leu Ile Leu Asp Phe Leu
                245                 250                 255

Gly Ile Ala Trp Ser Asp Ala Val Leu His His Glu Asp Leu Ile Gly
            260                 265                 270

Lys Pro Gly Gly Val Ser Leu Ser Lys Ile Glu Arg Ser Thr Asp Gln
        275                 280                 285

Val Ile Lys Pro Val Asn Leu Glu Ala Leu Ser Lys Trp Thr Gly His
    290                 295                 300

Ile Pro Gly Asp Val Val Arg Asp Met Ala Gln Ile Ala Pro Met Leu
305                 310                 315                 320

Ala Gln Leu Gly Tyr Asp Pro Tyr Ala Asn Pro Pro Asn Tyr Gly Asn
                325                 330                 335

Pro Asp Pro Phe Val Ile Asn Asn Thr Gln Arg Val Leu Lys Gly Asp
            340                 345                 350

Tyr Lys Thr Pro Ala Asn Leu Lys Gly Tyr Phe Gln Val Asn Gln Asn
        355                 360                 365

Ser Thr Ser Ser His Leu Gly Ser Ser
    370                 375

<210>  14
<211>  1189
<212>  DNA
<213>  Homo sapiens

<400>  14
atgcgcctgt cggtgcggag ggtgctgctg gcagccggct gcgccctggt cctggtgctg      60

gcggttcagc tgggacagca ggtgctagag tgccgggcgg tgctggcggg cctgcggagc     120

ccccgggggg ccatgcggcc tgagcaggag gagctggtga tggtgggcac caaccacgtg     180

gaataccgct atggcaaggc catgccgctc atcttcgtgg gtggcgtgcc tcgcagtggc     240

accacgttga tgcgcgccat gctggacgcc caccccgagg tgcgctgcgg cgaggagacc     300

cgcatcatcc cgcgcgtgct ggccatgcgc caggcctggt ccaagtctgg ccgtgagaag     360

ctgcggctgg atgaggcggg ggtgacggat gaggtgctgg acgccgccat gcaggccttc 420

atcctggagg tgattgccaa gcacggagag ccggcccgcg tgctctgcaa caaggaccca 480

tttacgctca agtcctcggt ctacctgtcg cgcctgttcc ccaactccaa gttcctgctg 540

atggtgcggg acggccgggc ctccgtgcac tccatgatca cgcgcaaagt caccattgcg 600

ggctttgacc tcagcagcta ccgtgactgc ctcaccaagt ggaacaaggc catcgaggtg 660

atgtacgccc agtgcatgga ggtaggcaag gagaagtgct tgcctgtgta ctacgagcag 720

ctggtgctgc accccaggcg ctcactcaag ctcatcctcg acttcctcgg catcgcctgg 780

agcgacgctg tcctccacca tgaagacctc attggcaagc ccggtggtgt ctccctgtcc 840

aagatcgagc ggtccacgga ccaggtcatc aagcctgtta acctggaagc gctctccaag 900

tggactggcc acatccctgg ggatgtggtg cgggacatgg cccagatcgc ccccatgctg 960

gctcagctcg gctatgaccc ttatgcaaac ccccccaact atggcaaccc tgaccccttc 1020

gtcatcaaca acacacagcg ggtcttgaaa ggggactata aaacaccagc caatctgaaa 1080

ggatattttc aggtgaacca gaacagcacc tcctcccact taggaagctc gtgatttcca 1140

gatctccgca aatgacttca ttgccaagaa gagaagaaaa tgcatttaa 1189

<210> 15
<211> 370
<212> PRT
<213> Mus musculus

<400> 15

Met Val Gly Lys Leu Lys Gln Asn Leu Leu Leu Ala Cys Leu Val Ile
1               5                   10                  15

Ser Ser Val Thr Val Phe Tyr Leu Gly Gln His Ala Met Glu Cys His
            20                  25                  30

His Arg Ile Glu Glu Arg Ser Gln Pro Ala Arg Leu Glu Asn Pro Lys
        35                  40                  45

Ala Thr Val Arg Ala Gly Leu Asp Ile Lys Ala Asn Lys Thr Phe Thr
    50                  55                  60

Tyr His Lys Asp Met Pro Leu Ile Phe Ile Gly Gly Val Pro Arg Ser
65                  70                  75                  80

Gly Thr Thr Leu Met Arg Ala Met Leu Asp Ala His Pro Asp Ile Arg
                85                  90                  95

Cys Gly Glu Glu Thr Arg Val Ile Pro Arg Ile Leu Ala Leu Lys Gln
            100                 105                 110

Met Trp Ser Arg Ser Ser Lys Glu Lys Ile Arg Leu Asp Glu Ala Gly
        115                 120                 125

Val Thr Asp Glu Val Leu Asp Ser Ala Met Gln Ala Phe Leu Leu Glu
    130                 135                 140

Val Ile Val Lys His Gly Glu Pro Ala Pro Tyr Leu Cys Asn Lys Asp
145                 150                 155                 160

Pro Phe Ala Leu Lys Ser Leu Thr Tyr Leu Ala Arg Leu Phe Pro Asn
                165                 170                 175

Ala Lys Phe Leu Leu Met Val Arg Asp Gly Arg Ala Ser Val His Ser
            180                 185                 190

Met Ile Ser Arg Lys Val Thr Ile Ala Gly Phe Asp Leu Asn Ser Tyr
        195                 200                 205

Arg Asp Cys Leu Thr Lys Trp Asn Arg Ala Ile Glu Thr Met Tyr Asn
    210                 215                 220

Gln Cys Met Glu Val Gly Tyr Lys Lys Cys Met Leu Val His Tyr Glu
225                 230                 235                 240

Gln Leu Val Leu His Pro Glu Arg Trp Met Arg Thr Leu Leu Lys Phe
                245                 250                 255

Leu His Ile Pro Trp Asn His Ser Val Leu His His Glu Glu Met Ile
            260                 265                 270

Gly Lys Ala Gly Gly Val Ser Leu Ser Lys Val Glu Arg Ser Thr Asp
        275                 280                 285

Gln Val Ile Lys Pro Val Asn Val Gly Ala Leu Ser Lys Trp Val Gly
    290                 295                 300

Lys Ile Pro Pro Asp Val Leu Gln Asp Met Ala Val Ile Ala Pro Met
305                 310                 315                 320

Leu Ala Lys Leu Gly Tyr Asp Pro Tyr Ala Asn Pro Pro Asn Tyr Gly
                325                 330                 335

Lys Pro Asp Pro Lys Ile Leu Glu Asn Thr Arg Arg Val Tyr Lys Gly
            340                 345                 350

Glu Phe Gln Leu Pro Asp Phe Leu Lys Glu Lys Pro Gln Thr Glu Gln

355                 360                 365

Val Glu
    370

<210>  16
<211>  390
<212>  PRT
<213>  Mus musculus

<400>  16

Met Arg Arg Ala Pro Trp Leu Gly Leu Arg Pro Trp Leu Gly Met Arg
1               5                   10                  15

Leu Ser Val Arg Lys Val Leu Leu Ala Ala Gly Cys Ala Leu Ala Leu
            20                  25                  30

Val Leu Ala Val Gln Leu Gly Gln Gln Val Leu Glu Cys Arg Ala Val
        35                  40                  45

Leu Gly Gly Thr Arg Asn Pro Arg Arg Met Arg Pro Glu Gln Glu Glu
    50                  55                  60

Leu Val Met Leu Gly Ala Asp His Val Glu Tyr Arg Tyr Gly Lys Ala
65                  70                  75                  80

Met Pro Leu Ile Phe Val Gly Gly Val Pro Arg Ser Gly Thr Thr Leu
                85                  90                  95

Met Arg Ala Met Leu Asp Ala His Pro Glu Val Arg Cys Gly Glu Glu
            100                 105                 110

Thr Arg Ile Ile Pro Arg Val Leu Ala Met Arg Gln Ala Trp Thr Lys
        115                 120                 125

Ser Gly Arg Glu Lys Leu Arg Leu Asp Glu Ala Gly Val Thr Asp Glu
    130                 135                 140

Val Leu Asp Ala Ala Met Gln Ala Phe Ile Leu Glu Val Ile Ala Lys
145                 150                 155                 160

His Gly Glu Pro Ala Arg Val Leu Cys Asn Lys Asp Pro Phe Thr Leu
                165                 170                 175

Lys Ser Ser Val Tyr Leu Ala Arg Leu Phe Pro Asn Ser Lys Phe Leu
            180                 185                 190

Leu Met Val Arg Asp Gly Arg Ala Ser Val His Ser Met Ile Thr Arg

195                 200                 205

Lys Val Thr Ile Ala Gly Phe Asp Leu Ser Ser Tyr Arg Asp Cys Leu
    210                 215                 220

Thr Lys Trp Asn Lys Ala Ile Glu Val Met Tyr Ala Gln Cys Met Glu
225                 230                 235                 240

Val Gly Arg Asp Lys Cys Leu Pro Val Tyr Tyr Glu Gln Leu Val Leu
                245                 250                 255

His Pro Arg Arg Ser Leu Lys Arg Ile Leu Asp Phe Leu Gly Ile Ala
            260                 265                 270

Trp Ser Asp Thr Val Leu His His Glu Asp Leu Ile Gly Lys Pro Gly
        275                 280                 285

Gly Val Ser Leu Ser Lys Ile Glu Arg Ser Thr Asp Gln Val Ile Lys
    290                 295                 300

Pro Val Asn Leu Glu Ala Leu Ser Lys Trp Thr Gly His Ile Pro Arg
305                 310                 315                 320

Asp Val Val Arg Asp Met Ala Gln Ile Ala Pro Met Leu Ala Arg Leu
                325                 330                 335

Gly Tyr Asp Pro Tyr Ala Asn Pro Pro Asn Tyr Gly Asn Pro Asp Pro
            340                 345                 350

Ile Val Ile Asn Asn Thr His Arg Val Leu Lys Gly Asp Tyr Lys Thr
        355                 360                 365

Pro Ala Asn Leu Lys Gly Tyr Phe Gln Val Asn Gln Asn Ser Thr Ser
    370                 375                 380

Pro His Leu Gly Ser Ser
385                 390

<210>  17
<211>  1113
<212>  DNA
<213>  Mus musculus

<400>  17
atggttggga agctgaagca gaacttactc ttggcgtgtc tggtgattag ttctgtgacc      60

gtgttttacc tgggccagca tgccatggag tgccatcacc gaatagagga acgtagccag     120

ccagcccgac tggagaaccc caaggcgact gtgcgagctg gcctcgacat caaagccaac     180

aaaacattca cctatcacaa agatatgcct ttaatattca tcgggggtgt gcctcggagc 240

ggcaccacac tcatgagggc tatgctggac gcacatcctg acatccgctg tggagaggaa 300

accagggtca tccctcgaat cctggccctg aagcagatgt ggtcccggtc cagtaaagag 360

aagatccgct tggatgaggc gggtgtcaca gatgaagtgc tagattctgc catgcaagcc 420

ttccttctgg aggtcattgt taaacatggg gagccggcac cttatttatg taacaaagat 480

ccgtttgccc tgaaatcctt gacttacctt gctaggttat ttcccaatgc caaatttctc 540

ctgatggtcc gagatggccg ggcgtcagta cattcaatga tttctcggaa agttactata 600

gctggctttg acctgaacag ctaccgggac tgtctgacca agtggaaccg ggccatagaa 660

accatgtaca accagtgtat ggaagttggt tataagaaat gcatgttggt tcactatgaa 720

cagctcgtct tacaccctga acggtggatg agaacgctct taaagttcct ccatattcca 780

tggaaccatt ccgttttgca ccatgaagaa atgatcggga aagctggggg agtttctctg 840

tcaaaggtgg aaagatcaac agaccaagtc atcaaacccg tcaacgtggg ggcgctatcg 900

aagtgggttg ggaagatacc cccggacgtc ttacaagaca tggccgtgat tgcacccatg 960

ctcgccaagc ttggatatga cccatacgcc aatcctccta actacggaaa acctgacccc 1020

aagatccttg aaaacaccag gagggtctat aaaggagaat ttcagctccc tgactttctg 1080

aaagaaaaac cccagacgga gcaagtggag taa 1113

<210> 18
<211> 1173
<212> DNA
<213> Mus musculus

<400> 18
atgaggcggg ccccctggct gggcctgcga ccctggctgg gcatgcgcct gtcggtgcgt 60

aaggtgctgc tggccgccgg ctgtgctctg gccctggtgc tcgctgtgca gcttgggcag 120

caagtactgg agtgccgggc ggtgctcggg ggcacacgga acccacggag gatgcggccg 180

gagcaggagg aactggtgat gctcggcgcc gaccacgtgg agtaccgcta tggcaaggcc 240

atgccactca tctttgtggg cggcgtgcca cgcagtggca ccacgctcat gcgcgccatg 300

ttggacgcac acccagaggt gcgctgtggg gaggagacgc gcatcatccc tcgtgtgctg 360

gccatgcggc aggcctggac caagtctggc cgtgagaagc tgcggctgga cgaggcaggt 420

gtgacggatg aggtgctgga cgcggccatg caggccttca ttctggaggt gatcgccaag 480

cacggcgaac cagcccgcgt gctgtgtaac aaggacccct tcacactcaa gtcatccgtc 540

tacctggcac gcctgttccc caactccaaa ttcctgctaa tggtgcgtga cggccgggcg 600

tccgtgcact ccatgatcac gcgcaaggtc accatcgcgg gctttgacct cagcagctac 660

cgagactgcc tcaccaagtg gaacaaggcc atcgaggtga tgtacgcaca gtgcatggag 720

gtgggcaggg acaagtgcct gcccgtgtac tatgagcagt tggtgctgca cccccggcgc 780

tcactcaaac gcatcctgga cttcctgggc atcgcctgga gtgacacagt cctgcaccac 840

gaggacctca ttggcaagcc tgggggcgtc tccttgtcca agatcgagcg gtccacggac 900

caggtcatca aaccggtgaa cttggaagct ctctccaagt ggacgggcca catccctaga 960

gacgtggtga gggatatggc ccagattgcc cccatgctgg cccggcttgg ctatgacccg 1020

tatgcgaatc cacccaacta tgggaacccc gaccccattg tcatcaacaa cacacaccgg 1080

gtcttgaaag gagactataa aacgccagcc aatctgaaag gatattttca ggtgaaccag 1140

aacagcacct ccccacacct aggaagttcg tga 1173

<210> 19
<211> 380
<212> PRT
<213> Caenorhabditis elegans

<400> 19

Met Arg Lys Asn Arg Glu Leu Leu Leu Val Leu Phe Leu Val Val Phe
1               5                   10                  15

Ile Leu Phe Tyr Phe Ile Thr Ala Arg Thr Ala Asp Asp Pro Tyr Tyr
            20                  25                  30

Ser Asn His Arg Glu Lys Phe Asn Gly Ala Ala Ala Asp Asp Gly Asp
        35                  40                  45

Glu Ser Leu Pro Phe His Gln Leu Thr Ser Val Arg Ser Asp Asp Gly
    50                  55                  60

Tyr Asn Arg Thr Ser Pro Phe Ile Phe Ile Gly Gly Val Pro Arg Ser
65                  70                  75                  80

Gly Thr Thr Leu Met Arg Ala Met Leu Asp Ala His Pro Glu Val Arg
                85                  90                  95

Cys Gly Glu Glu Thr Arg Val Ile Pro Arg Ile Leu Asn Leu Arg Ser
            100                 105                 110

Gln Trp Lys Lys Ser Glu Lys Glu Trp Asn Arg Leu Gln Gln Ala Gly
        115                 120                 125

Val Thr Gly Glu Val Ile Asn Asn Ala Ile Ser Ser Phe Ile Met Glu
    130                 135                 140

Ile Met Val Gly His Gly Asp Arg Ala Pro Arg Leu Cys Asn Lys Asp
145                 150                 155                 160

Pro Phe Thr Met Lys Ser Ala Val Tyr Leu Lys Glu Leu Phe Pro Asn
                165                 170                 175

Ala Lys Tyr Leu Leu Met Ile Arg Asp Gly Arg Ala Thr Val Asn Ser
            180                 185                 190

Ile Ile Ser Arg Lys Val Thr Ile Thr Gly Phe Asp Leu Asn Asp Phe
        195                 200                 205

Arg Gln Cys Met Thr Lys Trp Asn Ala Ala Ile Gln Ile Met Val Asp
    210                 215                 220

Gln Cys Glu Ser Val Gly Glu Lys Asn Cys Leu Lys Val Tyr Tyr Glu
225                 230                 235                 240

Gln Leu Val Leu His Pro Glu Ala Gln Met Arg Arg Ile Thr Glu Phe
                245                 250                 255

Leu Asp Ile Pro Trp Asp Asp Lys Val Leu His His Glu Gln Leu Ile
            260                 265                 270

Gly Lys Asp Ile Ser Leu Ser Asn Val Glu Arg Ser Ser Asp Gln Val
        275                 280                 285

Val Lys Pro Val Asn Leu Asp Ala Leu Ile Lys Trp Val Gly Thr Ile
    290                 295                 300

Pro Glu Asp Val Val Ala Asp Met Asp Ser Val Ala Pro Met Leu Arg
305                 310                 315                 320

Arg Leu Gly Tyr Asp Pro Asn Ala Asn Pro Pro Asn Tyr Gly Lys Pro
                325                 330                 335

Asp Glu Leu Val Ala Lys Lys Thr Glu Asp Val His Lys Asn Gly Ala
            340                 345                 350

Glu Trp Tyr Lys Lys Ala Val Gln Val Val Asn Asp Pro Gly Arg Val
        355                 360                 365

Asp Lys Pro Ile Val Asp Asn Glu Val Ser Lys Leu
    370                 375                 380

<210>  20
<211>  259
<212>  PRT
<213>  Caenorhabditis elegans

<400>  20

Met Arg Ala Ile Leu Asp Ala His Pro Asp Val Arg Cys Gly Gly Glu
1               5                   10                  15

Thr Met Leu Leu Pro Ser Phe Leu Thr Trp Gln Ala Gly Trp Arg Asn
            20                  25                  30

Asp Trp Val Asn Asn Ser Gly Ile Thr Gln Glu Val Phe Asp Asp Ala
        35                  40                  45

Val Ser Ala Phe Ile Thr Glu Ile Val Ala Lys His Ser Glu Leu Ala
    50                  55                  60

Pro Arg Leu Cys Asn Lys Asp Pro Tyr Thr Ala Leu Trp Leu Pro Thr
65                  70                  75                  80

Ile Arg Arg Leu Tyr Pro Asn Ala Lys Phe Ile Leu Met Ile Arg Asp
                85                  90                  95

Ala Arg Ala Val Val His Ser Met Ile Glu Arg Lys Val Pro Val Ala
            100                 105                 110

Gly Tyr Asn Thr Ser Asp Glu Ile Ser Met Phe Val Gln Trp Asn Gln
        115                 120                 125

Glu Leu Arg Lys Met Thr Phe Gln Cys Asn Asn Ala Pro Gly Gln Cys
    130                 135                 140

Ile Lys Val Tyr Tyr Glu Arg Leu Ile Gln Lys Pro Ala Glu Glu Ile
145                 150                 155                 160

Leu Arg Ile Thr Asn Phe Leu Asp Leu Pro Phe Ser Gln Gln Met Leu
                165                 170                 175

Arg His Gln Asp Leu Ile Gly Asp Glu Val Asp Leu Asn Asp Gln Glu
            180                 185                 190

Phe Ser Ala Ser Gln Val Lys Asn Ser Ile Asn Thr Lys Ala Leu Thr
        195                 200                 205

Ser Trp Phe Asp Cys Phe Ser Glu Glu Thr Leu Arg Lys Leu Asp Asp
    210                 215                 220

Val Ala Pro Phe Leu Gly Ile Leu Gly Tyr Asp Thr Ser Ile Ser Lys
225                 230                 235                 240

Pro Asp Tyr Ser Thr Phe Ala Asp Asp Asp Phe Tyr Gln Phe Lys Asn
                245                 250                 255

Phe Tyr Ser

<210> 21
<211> 1143
<212> DNA
<213> Caenorhabditis elegans

<400> 21
atgagaaaaa atcgagagtt gctactcgtc ctcttcctcg tcgtttttat actattctat 60

tttattactg cgagaactgc agacgacccg tactacagta accatcggga gaaattcaat 120

ggtgccgccg ccgacgacgg cgacgagtcg ttaccttttc atcaattaac gtcagtacga 180

agtgatgatg gatacaatag aacgtctcct ttcatattca taggtggtgt tcctcgctcc 240

ggtacaactc tgatgcgtgc gatgcttgac gctcatccag aagtcagatg tggtgaggag 300

acacgtgtca ttccacgcat cctgaatcta cggtcacaat ggaaaaagtc ggaaaaggag 360

tggaatcgac tgcagcaggc tggagtgacg ggtgaagtga ttaacaatgc gatcagctcg 420

tttatcatgg agataatggt tggccacgga gatcgggctc ctcgtctctg caacaaggat 480

ccattcacaa tgaaatcagc cgtctaccta aaagaactct tcccaaatgc caaatatctt 540

ctaatgatcc gtgatggacg ggccaccgtg aatagtataa tctcacgaaa agtcacaatt 600

accggattcg atttgaacga tttccgtcaa tgcatgacga aatggaatgc ggcaattcaa 660

ataatggtag atcagtgtga atcggttgga gagaaaaatt gtttgaaagt gtattatgag 720

cagctggtgc tacatccgga agcacaaatg cggcgaatta cagagttttt ggatattccg 780

tgggatgata aagtgctgca ccatgagcag cttattggaa aagatatttc tttatcgaat 840

gtggaacgga gctcggatca agtcgttaaa ccggttaatc ttgatgctct tatcaaatgg 900

gttggaacga ttcctgagga tgttgttgct gatatggatt cggttgcgcc gatgttaagg 960

agattaggat atgatccgaa tgcaaatcca ccaaactatg gaaaacccga cgaactagtc 1020

gcgaaaaaaa cggaagatgt tcataaaaat ggagccgaat ggtacaagaa agcagttcaa 1080

gtggtcaacg atcccggccg cgtcgataaa ccaattgttg ataatgaagt atcgaaatta 1140

tag 1143

<210> 22
<211> 780
<212> DNA
<213> Caenorhabditis elegans

<400> 22
atgagagcta ttctagatgc acatccggat gttcgatgtg gcggtgaaac catgctgctt 60

ccaagtttcc ttacatggca agcaggctgg cggaatgatt gggtcaataa ttcaggaatt 120

actcaggaag tatttgacga cgctgtttca gcattcatca ctgagatagt cgcgaagcac 180

agtgaactag cacctcgtct gtgcaacaag gatccataca ccgcattgtg gcttccgact 240

attcgccgac tgtacccgaa tgcaaagttt attctgatga ttcgagatgc tcgtgccgta 300

gttcattcaa tgatagaaag aaaagtacca gttgctgggt ataatacgtc tgatgaaatt 360

tcaatgtttg ttcagtggaa tcaggagctt cgaaaaatga cttttcaatg caataatgcg 420

ccagggcaat gcataaaagt atattatgaa cgactgattc aaaaacctgc ggaagaaatc 480

ctacgtatca ccaacttcct ggatctgcca ttttcccagc aaatgctaag acatcaagat 540

ttaattggag acgaagttga tttaaacgat caagaattct ctgcatcaca agttaaaaac 600

tcgataaaca ctaaagcctt aacctcgtgg tttgattgtt ttagtgaaga aactctacga 660

aaacttgatg acgtggcacc ttttttggga attcttggat acgatacgtc gatttcaaaa 720

cccgattatt ccacatttgc ggatgacgat ttttaccaat ttaaaaattt ttattcttaa 780

<210> 23
<211> 499
<212> PRT
<213> Drosophila melanogaster

<400> 23

Met Arg Leu Pro Tyr Arg Asn Lys Lys Val Thr Leu Trp Val Leu Phe
1               5                   10                  15

Gly Ile Ile Val Ile Thr Met Phe Leu Phe Lys Phe Thr Glu Leu Arg
            20                  25                  30

Pro Thr Cys Leu Phe Lys Val Asp Ala Ala Asn Glu Leu Ser Ser Gln
        35                  40                  45

Met Val Arg Val Glu Lys Tyr Leu Thr Asp Asp Asn Gln Arg Val Tyr
    50                  55                  60

Ser Tyr Asn Arg Glu Met Pro Leu Ile Phe Ile Gly Gly Val Pro Arg
65                  70                  75                  80

Ser Gly Thr Thr Leu Met Arg Ala Met Leu Asp Ala His Pro Asp Val
                85                  90                  95

Arg Cys Gly Gln Glu Thr Arg Val Ile Pro Arg Ile Leu Gln Leu Arg
            100                 105                 110

Ser His Trp Leu Lys Ser Glu Lys Glu Ser Leu Arg Leu Gln Glu Ala
        115                 120                 125

Gly Ile Thr Lys Glu Val Met Asn Ser Ala Ile Ala Gln Phe Cys Leu

130                 135                 140

Glu Ile Ile Ala Lys His Gly Glu Pro Ala Pro Arg Leu Cys Asn Lys
145                 150                 155                 160

Asp Pro Leu Thr Leu Lys Met Gly Ser Tyr Val Ile Glu Leu Phe Pro
                165                 170                 175

Asn Ala Lys Phe Leu Phe Met Val Arg Asp Gly Arg Ala Thr Val His
            180                 185                 190

Ser Ile Ile Ser Arg Lys Val Thr Ile Thr Gly Phe Asp Leu Ser Ser
        195                 200                 205

Tyr Arg Gln Cys Met Gln Lys Trp Asn His Ala Ile Glu Val Met His
    210                 215                 220

Glu Gln Cys Arg Asp Ile Gly Lys Asp Arg Cys Met Met Val Tyr Tyr
225                 230                 235                 240

Glu Gln Leu Val Leu His Pro Glu Glu Trp Met Arg Lys Ile Leu Lys
                245                 250                 255

Phe Leu Asp Val Pro Trp Asn Asp Ala Val Leu His His Glu Glu Phe
            260                 265                 270

Ile Asn Lys Pro Asn Gly Val Pro Leu Ser Lys Val Glu Arg Ser Ser
        275                 280                 285

Asp Gln Val Ile Lys Pro Val Asn Leu Glu Ala Met Ser Lys Trp Val
    290                 295                 300

Gly Gln Ile Pro Gly Asp Val Val Arg Asp Met Ala Asp Ile Ala Pro
305                 310                 315                 320

Met Leu Ser Val Leu Gly Tyr Asp Pro Tyr Ala Asn Pro Pro Asp Tyr
                325                 330                 335

Gly Lys Pro Asp Ala Trp Val Gln Asp Asn Thr Ser Lys Leu Lys Ala
            340                 345                 350

Asn Arg Met Leu Trp Glu Ser Lys Ala Lys Gln Val Leu Gln Met Ser
        355                 360                 365

Ser Ser Glu Asp Asp Asn Thr Asn Thr Ile Ile Asn Asn Ser Asn Asn
    370                 375                 380

Lys Asp Asn Asn Asn Asn Gln Tyr Thr Ile Asn Lys Ile Ile Pro Glu
385                 390                 395                 400

Gln His Ser Arg Gln Arg Gln His Val Gln Gln Gln His Leu Gln Gln
                405                 410                 415

Gln Gln Gln Gln His Leu Gln Gln Gln Gln His Gln Arg Gln Gln Gln
            420                 425                 430

Gln Gln Gln Arg Glu Glu Glu Ser Glu Ser Glu Arg Glu Ala Glu Pro
        435                 440                 445

Asp Arg Glu Gln Gln Leu Leu His Gln Lys Pro Lys Asp Val Ile Thr
    450                 455                 460

Ile Lys Gln Leu Pro Leu Ala Gly Ser Asn Asn Asn Asn Ile Asn Asn
465                 470                 475                 480

Asn Ile Asn Asn Asn Asn Asn Asn Asn Asn Ile Met Glu Asp Pro Met
                485                 490                 495

Ala Asp Thr

<210>  24
<211>  1500
<212>  DNA
<213>  Drosophila melanogaster

<400>  24
atgcgactgc catatcgaaa taagaaggtc accctgtggg tgctcttcgg catcatcgtc      60

atcaccatgt tcctattcaa attcaccgaa ctgcggccca catgcctctt caaggtggac     120

gccgccaacg agctctcctc ccaaatggtt cgcgttgaga aatacctcac agatgacaat     180

caacgcgttt attcatacaa ccgtgagatg ccattaatat tcataggcgg cgtgccgaga     240

tctgggacga ctttgatgcg cgccatgctg gatgcccatc ccgatgtgcg ctgcgggcag     300

gaaacccgtg tcattccgcg catcctgcag ctgcgctcgc actggctgaa gtccgagaag     360

gagtcgctcc gcctgcagga ggccggcatc accaaagagg tcatgaacag tgccatcgcg     420

cagttctgtc tggaaatcat cgccaaacac ggcgagccgg cgccgcgctt atgcaacaag     480

gatccgctga cgctgaaaat gggctcctat gtcatcgagc tatttccgaa cgctaaattc     540

ctattcatgg tgcgcgacgg ccgggcgaca gttcattcga ttatatcgcg caaggtgaca     600

atcaccggct tcgatttgag cagctaccgg cagtgcatgc agaagtggaa ccacgccatc     660

gaggtgatgc acgagcagtg ccgggacatc ggcaaggacc gctgcatgat ggtttactat     720

gagcagctgg tactgcatcc cgaggagtgg atgcgaaaga tactgaaatt cctggacgtg     780

ccatggaacg atgcggtgct gcaccacgag gagttcataa ataaaccgaa cggtgtgcct 840

ctgtccaagg tggaacgttc gtcggaccag gttatcaagc cggttaatct ggaggcgatg 900

tccaaatggg ttggccaaat acccggcgac gtggtgcgcg acatggccga catagcgccc 960

atgctgtccg tgctcggcta cgatccgtac gcgaatccgc cggactatgg taagccagat 1020

gcatgggtgc aggacaacac gtcgaagtta aaggccaatc gaatgctgtg ggagagtaag 1080

gcgaagcaag tgctgcagat gtcatccagc gaggatgaca acacgaacac catcatcaac 1140

aatagcaaca ataaggataa caacaataat cagtacacaa tcaataaaat tataccagaa 1200

caacacagca gacagcggca acatgtacag cagcaacatc tgcagcagca gcagcagcag 1260

catctgcaac agcagcaaca tcagcggcag cagcaacagc agcaacgtga ggaggagagc 1320

gagtcggaaa gggaagcgga accggatcga gaacaacaat tgttgcatca aaagccaaag 1380

gatgtcatta cgataaagca gctgccatta gctgggagca acaataacaa catcaacaat 1440

aacatcaaca acaacaacaa caacaacaac atcatggagg accccatggc ggatacatga 1500

<210> 25
<211> 500
<212> PRT
<213> Arabidopsis thaliana

<400> 25

Met Gln Met Asn Ser Val Trp Lys Leu Ser Leu Gly Leu Leu Leu Leu
1               5                   10                  15

Ser Ser Val Ile Gly Ser Phe Ala Glu Leu Asp Phe Gly His Cys Glu
            20                  25                  30

Thr Leu Val Lys Lys Trp Ala Asp Ser Ser Ser Ser Arg Glu Glu His
        35                  40                  45

Val Asn Lys Asp Lys Arg Ser Leu Lys Asp Leu Leu Phe Phe Leu His
    50                  55                  60

Val Pro Arg Thr Gly Gly Arg Thr Tyr Phe His Cys Phe Leu Arg Lys
65                  70                  75                  80

Leu Tyr Asp Ser Ser Glu Glu Cys Pro Arg Ser Tyr Asp Lys Leu His
                85                  90                  95

Phe Asn Pro Arg Lys Glu Lys Cys Lys Leu Leu Ala Thr His Asp Asp
            100                 105                 110

Tyr Ser Leu Met Ala Lys Leu Pro Arg Glu Arg Thr Ser Val Met Thr
        115                 120                 125

Ile Val Arg Asp Pro Ile Ala Arg Val Leu Ser Thr Tyr Glu Phe Ser
    130                 135                 140

Val Glu Val Ala Ala Arg Phe Leu Val His Pro Asn Leu Thr Ser Ala
145                 150                 155                 160

Ser Arg Met Ser Ser Arg Ile Arg Lys Ser Asn Val Ile Ser Thr Leu
                165                 170                 175

Asp Ile Trp Pro Trp Lys Tyr Leu Val Pro Trp Met Arg Glu Asp Leu
            180                 185                 190

Phe Ala Arg Arg Asp Ala Arg Lys Leu Lys Glu Val Val Ile Ile Glu
        195                 200                 205

Asp Asp Asn Pro Tyr Asp Met Glu Glu Met Leu Met Pro Leu His Lys
    210                 215                 220

Tyr Leu Asp Ala Pro Thr Ala His Asp Ile Ile His Asn Gly Ala Thr
225                 230                 235                 240

Phe Gln Ile Ala Gly Leu Thr Asn Asn Ser His Leu Ser Glu Ala His
                245                 250                 255

Glu Val Arg His Cys Val Gln Lys Phe Lys Ser Leu Gly Glu Ser Val
            260                 265                 270

Leu Gln Val Ala Lys Arg Arg Leu Asp Ser Met Leu Tyr Val Gly Leu
        275                 280                 285

Thr Glu Glu His Arg Glu Ser Ala Ser Leu Phe Ala Asn Val Val Gly
    290                 295                 300

Ser Gln Val Leu Ser Gln Val Val Pro Ser Asn Ala Thr Ala Lys Ile
305                 310                 315                 320

Lys Ala Leu Lys Ser Glu Ala Ser Val Thr Ile Ser Glu Thr Gly Ser
                325                 330                 335

Asp Lys Ser Asn Ile Gln Asn Gly Thr Ser Glu Val Thr Leu Asn Lys
            340                 345                 350

Ala Glu Ala Lys Ser Gly Asn Met Thr Val Lys Thr Leu Met Glu Val
        355                 360                 365

Tyr Glu Gly Cys Ile Thr His Leu Arg Lys Ser Gln Gly Thr Arg Arg

370                 375                 380

Val Asn Ser Leu Lys Arg Ile Thr Pro Ala Asn Phe Thr Arg Gly Thr
385                 390                 395                 400

Arg Thr Arg Val Pro Lys Glu Val Ile Gln Gln Ile Lys Ser Leu Asn
                405                 410                 415

Asn Leu Asp Val Glu Leu Tyr Lys Tyr Ala Lys Val Ile Phe Ala Lys
            420                 425                 430

Glu His Glu Leu Val Ser Asn Lys Leu Ile Ser Ser Ser Lys Arg Ser
        435                 440                 445

Ile Val Asp Leu Pro Ser Glu Leu Lys Ser Val Leu Gly Glu Met Gly
    450                 455                 460

Glu Glu Lys Leu Trp Lys Phe Val Pro Val Ala Leu Met Leu Leu Leu
465                 470                 475                 480

Ile Val Leu Phe Phe Leu Phe Val Asn Ala Lys Arg Arg Arg Thr Ser
                485                 490                 495

Lys Val Lys Ile
            500

<210>  26
<211>  1503
<212>  DNA
<213>  Arabidopsis thaliana

<400>  26
atgcaaatga actctgtttg gaagctgtct cttgggttat tacttcttag ctcagttatt      60

ggctcttttg cggaacttga ttttggccat tgcgaaactc ttgtgaaaaa atgggctgat      120

tcttcttcat ctcgtgaaga acatgttaat aaagacaaac gctcgcttaa ggatttgctc      180

ttctttctcc acgttccgcg aactggaggc agaacatatt ttcattgttt tttgaggaag      240

ttgtatgata gctctgagga atgtcctcga tcttacgaca agctccactt caatccaagg      300

aaggaaaagt gcaagttgtt agccacacat gatgattata gtttgatggc aaagcttccg      360

agggagagaa cttcggtgat gacaatagtt cgggatccta ttgcgcgtgt gttaagcact      420

tatgaatttt ccgtagaggt agcagctagg tttttggtgc atcccaattt aacttctgcg      480

tcaaggatgt ctagccgcat acgcaagagt aatgtaataa gcacactaga catatggcca      540

tggaaatacc tagttccatg gatgagagaa gacttgtttg ctcggcgaga tgcacgaaaa      600

ttgaaggagg tagtgatcat tgaggacgat aacccgtatg acatggagga gatgcttatg      660

cctttgcaca aatatcttga tgcgcctact gctcatgaca tcatccacaa tggagcgact 720

tttcagattg caggattgac aaataactcc catttatcag aagcacacga ggttcggcat 780

tgtgtgcaga aattcaaaag ccttggtgag tctgttctcc aagttgccaa gaggaggcta 840

gacagcatgt tgtatgttgg actgacagag gagcacaggg aatctgcatc actttttgcc 900

aatgtagtgg gttctcaagt gctgtctcaa gtggttccgt ccaatgcaac tgcgaaaatc 960

aaagctctta aatcagaagc aagtgtcaca atttcagaaa ccgggtcaga taagagtaat 1020

attcagaatg gtacatctga agttacattg aataaggcag aagctaagag tgggaatatg 1080

acggtaaaaa cccttatgga agtctatgaa ggctgcatca ctcatttacg aaagtcccaa 1140

ggaaccagac gggtcaactc tctgaagaga ataactccag caaattttac aagagggacg 1200

cgtacaagag ttcctaaaga ggtcattcag cagatcaaat cgcttaacaa cctcgatgtg 1260

gagctctaca aatatgcaaa agtaatcttt gccaaagaac atgaattagt gtcgaataag 1320

ttgatctcaa gttctaagag aagcattgtt gatctgccga gtgagttaaa gagcgtattg 1380

ggagaaatgg gtgaagagaa gctatggaag ttcgtaccag tggcattgat gcttttattg 1440

atcgtcctct tctttctatt tgtaaacgct aaaaggagaa gaacctccaa agttaagatt 1500

tga 1503

<210> 27
<211> 23
<212> PRT
<213> Artificial Sequence

<220>
<223> PG9 fragment

<400> 27

Asn Gly Tyr Asn Tyr Tyr Asp Phe Tyr Asp Gly Tyr Tyr Asn Tyr His
1               5                   10                  15

Tyr Met Asp Val Trp Gly Lys
            20

<210> 28
<211> 6
<212> PRT
<213> Artificial Sequence

<220>
<223> PG9 fragment

<400> 28

Asn Gly Tyr Asn Tyr Tyr
1               5

<210>  29
<211>  12
<212>  PRT
<213>  Artificial Sequence

<220>
<223>  PG9 fragment

<400>  29

Asp Phe Tyr Asp Gly Tyr Tyr Asn Tyr His Tyr Met
1               5                   10


<210>  30
<211>  240
<212>  PRT
<213>  Artificial Sequence

<220>
<223>  PG9LC

<400>  30

Met Ala Asn Lys His Leu Ser Leu Ser Leu Phe Leu Val Leu Leu Gly
1               5                   10                  15

Leu Ser Ala Ser Leu Ala Ser Gly Gln Ser Ala Leu Thr Gln Pro Ala
            20                  25                  30

Ser Val Ser Gly Ser Pro Gly Gln Ser Ile Thr Ile Ser Cys Gln Gly
        35                  40                  45

Thr Ser Asn Asp Val Gly Gly Tyr Glu Ser Val Ser Trp Tyr Gln Gln
    50                  55                  60

His Pro Gly Lys Ala Pro Lys Val Val Ile Tyr Asp Val Ser Lys Arg
65                  70                  75                  80

Pro Ser Gly Val Ser Asn Arg Phe Ser Gly Ser Lys Ser Gly Asn Thr
                85                  90                  95

Ala Ser Leu Thr Ile Ser Gly Leu Gln Ala Glu Asp Glu Gly Asp Tyr
            100                 105                 110

Tyr Cys Lys Ser Leu Thr Ser Thr Arg Arg Arg Val Phe Gly Thr Gly
        115                 120                 125

Thr Lys Leu Thr Val Leu Gly Gln Pro Lys Ala Ala Pro Ser Val Thr
    130                 135                 140

Leu Phe Pro Pro Ser Ser Glu Glu Leu Gln Ala Asn Lys Ala Thr Leu
145                 150                 155                 160

Val Cys Leu Ile Ser Asp Phe Tyr Pro Gly Ala Val Thr Val Ala Trp
                165                 170                 175

Lys Ala Asp Ser Ser Pro Val Lys Ala Gly Val Glu Thr Thr Thr Pro
            180                 185                 190

Ser Lys Gln Ser Asn Asn Lys Tyr Ala Ala Ser Ser Tyr Leu Ser Leu
        195                 200                 205

Thr Pro Glu Gln Trp Lys Ser His Lys Ser Tyr Ser Cys Gln Val Thr
    210                 215                 220

His Glu Gly Ser Thr Val Glu Lys Thr Val Ala Pro Thr Glu Cys Ser
225                 230                 235                 240

<210>  31
<211>  723
<212>  DNA
<213>  Artificial Sequence

<220>
<223>  PG9LC

<400>  31
atggcgaaca aacacttgtc cctctccctc ttcctcgtcc tccttggcct gtcggccagc      60

ttggcctcag gtcagagtgc tcttactcag cctgcttctg tttctggttc tcctggtcag     120

agcatcacca tttcttgcca gggaacctct aacgatgtgg gaggttacga gtccgtgtct     180

tggtatcaac agcatcctgg taaggctcct aaggtggtga tctacgatgt gagcaagagg     240

ccttctggtg tgagcaatag gttcagcggt agcaagtctg gtaacaccgc ttctcttacc     300

atctctggac ttcaggctga ggatgaggga gattactact gcaagtctct gacctccact     360

agaagaaggg tgttcggaac cggtactaag cttactgttc tgggtcaacc taaggctgct     420

ccttctgtga ctttgttccc tccatcttct gaggaactgc aggctaacaa ggctaccctt     480

gtgtgcctga tcagcgattt ttaccctggt gctgttaccg tggcttggaa ggctgattct     540

tcacctgtta aggctggtgt ggaaaccacc actcctagca agcagagcaa caacaagtac     600

gctgctagct cctaccttag ccttactcct gaacagtgga agtcccacaa gagctactca     660

tgccaggtta cccatgaggg ttctaccgtg gaaaagactg ttgctcctac tgagtgcagc     720

tag                                                                   723

<210>  32
<211>  240
<212>  PRT
<213>  Artificial Sequence

<220>

<223>  PG9LC-RSH

<400>  32

Met Ala Asn Lys His Leu Ser Leu Ser Leu Phe Leu Val Leu Leu Gly
1               5                   10                  15

Leu Ser Ala Ser Leu Ala Ser Gly Gln Ser Ala Leu Thr Gln Pro Ala
            20                  25                  30

Ser Val Ser Gly Ser Pro Gly Gln Ser Ile Thr Ile Ser Cys Gln Gly
        35                  40                  45

Thr Ser Asn Asp Val Gly Gly Tyr Glu Ser Val Ser Trp Tyr Gln Gln
    50                  55                  60

His Pro Gly Lys Ala Pro Lys Val Val Ile Tyr Asp Val Ser Lys Arg
65                  70                  75                  80

Pro Ser Gly Val Ser Asn Arg Phe Ser Gly Ser Lys Ser Gly Asn Thr
                85                  90                  95

Ala Ser Leu Thr Ile Ser Gly Leu Gln Ala Glu Asp Glu Gly Asp Tyr
            100                 105                 110

Tyr Cys Lys Ser Leu Thr Ser Arg Ser His Arg Val Phe Gly Thr Gly
        115                 120                 125

Thr Lys Leu Thr Val Leu Gly Gln Pro Lys Ala Ala Pro Ser Val Thr
    130                 135                 140

Leu Phe Pro Pro Ser Ser Glu Glu Leu Gln Ala Asn Lys Ala Thr Leu
145                 150                 155                 160

Val Cys Leu Ile Ser Asp Phe Tyr Pro Gly Ala Val Thr Val Ala Trp
                165                 170                 175

Lys Ala Asp Ser Ser Pro Val Lys Ala Gly Val Glu Thr Thr Thr Pro
            180                 185                 190

Ser Lys Gln Ser Asn Asn Lys Tyr Ala Ala Ser Ser Tyr Leu Ser Leu
        195                 200                 205

Thr Pro Glu Gln Trp Lys Ser His Lys Ser Tyr Ser Cys Gln Val Thr
    210                 215                 220

His Glu Gly Ser Thr Val Glu Lys Thr Val Ala Pro Thr Glu Cys Ser
225                 230                 235                 240

<210> 33
<211> 723
<212> DNA
<213> Artificial Sequence

<220>
<223> PG9LC-RSH

<400> 33
atggcgaaca aacacttgtc cctctccctc ttcctcgtcc tccttggcct gtcggccagc 60

ttggcctcag gtcagagtgc tcttactcag cctgcttctg tttctggttc tcctggtcag 120

agcatcacca tttcttgcca gggaacctct aacgatgtgg gaggttacga gtccgtgtct 180

tggtatcaac agcatcctgg taaggctcct aaggtggtga tctacgatgt gagcaagagg 240

ccttctggtg tgagcaatag gttcagcggt agcaagtctg gtaacaccgc ttctcttacc 300

atctctggac ttcaggctga ggatgaggga gattactact gcaagtctct gacctccaga 360

agtcacaggg tgttcggaac cggtactaag cttactgttc tgggtcaacc taaggctgct 420

ccttctgtga ctttgttccc tccatcttct gaggaactgc aggctaacaa ggctaccctt 480

gtgtgcctga tcagcgattt ttaccctggt gctgttaccg tggcttggaa ggctgattct 540

tcacctgtta aggctggtgt ggaaaccacc actcctagca agcagagcaa caacaagtac 600

gctgctagct cctaccttag ccttactcct gaacagtgga agtcccacaa gagctactca 660

tgccaggtta cccatgaggg ttctaccgtg gaaaagactg ttgctcctac tgagtgcagc 720

tag 723

<210> 34
<211> 1392
<212> DNA
<213> Artificial Sequence

<220>
<223> gp120ZM109

<400> 34
atgcctatgg gcagcctgca gcccctggcc acactgtatc tgctgggaat gctggtggcc 60

agctgcctgg gcgtgtggaa agaggccaag accaccctgt tctgcgccag cgacgccaag 120

agctacgagc gcgaggtgca caatgtgtgg gccacccatg cctgcgtgcc caccgatcct 180

gatccccagg aactcgtgat ggccaacgtg accgagaact tcaacatgtg gaagaacgac 240

atggtggacc agatgcacga ggacatcatc agcctgtggg accagagcct gaagccctgc 300

gtgaagctga cccctctgtg cgtgaccctg aactgcacat ctcctgccgc ccacaacgag 360

agcgagacaa gagtgaagca ctgcagcttc aacatcacca ccgacgtgaa ggaccggaag 420

cagaaagtga acgccacctt ctacgacctg gacatcgtgc ccctgagcag cagcgacaac 480

agcagcaaca gctccctgta cagactgatc agctgcaaca ccagcaccat cacccaggcc 540

tgccccaagg tgtccttcga ccccatcccc atccactact gtgcccctgc cggctacgcc 600

atcctgaagt gcaacaacaa gaccttcagc ggcaagggcc cctgcagcaa cgtgtccacc 660

gtgcagtgta cccacggcat cagacccgtg gtgtccaccc agctgctgct gaatggcagc 720

ctggccgaag aggaaatcgt gatcagaagc gagaacctga ccgacaacgc caagacaatc 780

attgtgcatc tgaacaagag cgtggaaatc gagtgcatca ggcccggcaa caacaccaga 840

aagagcatca gactgggccc tggccagacc ttttacgcca ccggggatgt gatcggcgac 900

atccggaagg cctactgcaa gatcaacggc agcgagtgga acgagacact gacaaaggtg 960

tccgagaagc tgaaagagta ctttaacaag accattcgct tcgcccagca ctctggcggc 1020

gacctggaag tgaccaccca cagcttcaat tgcagaggcg agttcttcta ctgcaatacc 1080

agcgagctgt tcaacagcaa cgccaccgag agcaatatca ccctgccctg ccggatcaag 1140

cagatcatca atatgtggca gggcgtgggc agagctatgt acgcccctcc catccggggc 1200

gagatcaagt gcacctctaa catcaccggc ctgctgctga ccagggacgg cggaaacaac 1260

aacaatagca ccgaggaaat cttccggccc gagggcggca acatgagaga caattggaga 1320

tccgagctgt acaagtacaa ggtggtggaa atcaagggcc tgcggggcag ccaccaccat 1380

catcaccatt ga 1392

<210> 35
<211> 463
<212> PRT
<213> Artificial Sequence

<220>
<223> gp120ZM109

<400> 35

Met Pro Met Gly Ser Leu Gln Pro Leu Ala Thr Leu Tyr Leu Leu Gly
1               5                   10                  15

Met Leu Val Ala Ser Cys Leu Gly Val Trp Lys Glu Ala Lys Thr Thr
            20                  25                  30

Leu Phe Cys Ala Ser Asp Ala Lys Ser Tyr Glu Arg Glu Val His Asn
        35                  40                  45

Val Trp Ala Thr His Ala Cys Val Pro Thr Asp Pro Asp Pro Gln Glu
    50                  55                  60

Leu Val Met Ala Asn Val Thr Glu Asn Phe Asn Met Trp Lys Asn Asp
65                  70                  75                  80

Met Val Asp Gln Met His Glu Asp Ile Ile Ser Leu Trp Asp Gln Ser

85                  90                  95

Leu Lys Pro Cys Val Lys Leu Thr Pro Leu Cys Val Thr Leu Asn Cys
            100                 105                 110

Thr Ser Pro Ala Ala His Asn Glu Ser Glu Thr Arg Val Lys His Cys
        115                 120                 125

Ser Phe Asn Ile Thr Thr Asp Val Lys Asp Arg Lys Gln Lys Val Asn
    130                 135                 140

Ala Thr Phe Tyr Asp Leu Asp Ile Val Pro Leu Ser Ser Ser Asp Asn
145                 150                 155                 160

Ser Ser Asn Ser Ser Leu Tyr Arg Leu Ile Ser Cys Asn Thr Ser Thr
                165                 170                 175

Ile Thr Gln Ala Cys Pro Lys Val Ser Phe Asp Pro Ile Pro Ile His
            180                 185                 190

Tyr Cys Ala Pro Ala Gly Tyr Ala Ile Leu Lys Cys Asn Asn Lys Thr
        195                 200                 205

Phe Ser Gly Lys Gly Pro Cys Ser Asn Val Ser Thr Val Gln Cys Thr
    210                 215                 220

His Gly Ile Arg Pro Val Val Ser Thr Gln Leu Leu Leu Asn Gly Ser
225                 230                 235                 240

Leu Ala Glu Glu Glu Ile Val Ile Arg Ser Glu Asn Leu Thr Asp Asn
                245                 250                 255

Ala Lys Thr Ile Ile Val His Leu Asn Lys Ser Val Glu Ile Glu Cys
            260                 265                 270

Ile Arg Pro Gly Asn Asn Thr Arg Lys Ser Ile Arg Leu Gly Pro Gly
        275                 280                 285

Gln Thr Phe Tyr Ala Thr Gly Asp Val Ile Gly Asp Ile Arg Lys Ala
    290                 295                 300

Tyr Cys Lys Ile Asn Gly Ser Glu Trp Asn Glu Thr Leu Thr Lys Val
305                 310                 315                 320

Ser Glu Lys Leu Lys Glu Tyr Phe Asn Lys Thr Ile Arg Phe Ala Gln
                325                 330                 335

His Ser Gly Gly Asp Leu Glu Val Thr Thr His Ser Phe Asn Cys Arg
            340                 345                 350

Gly Glu Phe Phe Tyr Cys Asn Thr Ser Glu Leu Phe Asn Ser Asn Ala
        355                 360                 365

Thr Glu Ser Asn Ile Thr Leu Pro Cys Arg Ile Lys Gln Ile Ile Asn
    370                 375                 380

Met Trp Gln Gly Val Gly Arg Ala Met Tyr Ala Pro Pro Ile Arg Gly
385                 390                 395                 400

Glu Ile Lys Cys Thr Ser Asn Ile Thr Gly Leu Leu Leu Thr Arg Asp
                405                 410                 415

Gly Gly Asn Asn Asn Asn Ser Thr Glu Glu Ile Phe Arg Pro Glu Gly
            420                 425                 430

Gly Asn Met Arg Asp Asn Trp Arg Ser Glu Leu Tyr Lys Tyr Lys Val
        435                 440                 445

Val Glu Ile Lys Gly Leu Arg Gly Ser His His His His His His
    450                 455                 460